# EUROPEAN PATENT APPLICATION

(11) **EP 1 975 176 A1**
(43) Date of publication of application: **01.10.2008**
(21) Application number: 07006321.9
(22) Date of filing: 27.03.2007
(51) Int. Cl.: C07K 14/605, A61K 38/26, A61P 3/04, A61P 3/10, A61P 25/00, C07K 19/00

(54) **Novel glp-1 fusion peptides, their production and use**

(71) Applicant: Biocompatibles UK Limited, Farnham Surrey GU9 8QL (GB)
(72) Inventor: Wallrapp, Chritine,. Dr, 63762 Grossostheim (DE); Thoenes, Eric, 63654 Büdingen (DE); Geigle, Peter., Dr, 63755 Alzenau (DE)
(74) Representative: Graf von Stosch, Andreas

(57) **Abstract**

The present invention provides novel fusion peptides having GLP-1 activity and enhanced stability in vivo, in particular resistancy to dipeptidyl peptidase IV. The fusion peptide comprises as component (I) N-terminally a GLP-1(7-35, 7-36 or 7-37) sequence and as component (II) C-terminally a peptide sequence of at least 9 amino acids or a functional fragment, variant or derivative thereof. Component (II) is preferably a full or partial version of a homologue of native IP2 (intervening peptide 2). A preferred embodiment comprises the sequence GLP-1(7-35, 36 or 37)/IP2-homologue/GLP-1(7-35, 36 or 37) or GLP-2. The fusion peptide may be produced in engineered cells or synthetically and may be used for the preparation of a medicament for treating various diseases or disorders, e.g. diabetes type 1 or 2, apoptosis related diseases or neurodegenerative disorders.

## Description

The present invention relates to novel GLP-1 fusion peptides which have extended C termini, are resistant to endopeptidase IV inactivation, may be expressed at high levels in transformed animal cells, and are e.g. useful in the treatment of type 2 diabetes.

The glucagon gene is well studied, see e.g. White, J.W. et al., 1986 Nucleic Acid Res. 14(12) 4719-4730. The preproglucagon molecule as a high molecular weight precursor molecule is synthesised in pancreatic alpha cells and in the jejunum and colon L cells. Preproglucagon is a 180 amino acid long prohormone and its sequence contains, in addition to glucagon, two sequences of related structure: glucagon-like peptide-1 (GLP-1) and glucagon-like peptide-2 (GLP-2). In the preproglucagon molecule, between GLP-1 and GLP-2 is a 17 amino acid peptide sequence (or rather a 15 amino acid sequence plus the C-terminal RR cleavage site), intervening peptide 2 (IP2). The IP2 sequence (located between GLP-1 and -2 in the precursor molecule) is normally cleaved proteolytically after aa 37 of GLP-1. The preproglucagon module is therefore cleaved into various peptides, depending on the cell, and the environment, including GLP-1 (1-37), a 37 amino acid peptide in its unprocessed form. Generally, this processing occurs in the pancreas and the intestine. The GLP-1 (1-37) sequence can be further proteolytically processed into active GLP-1 (7-37), the 31 amino acid processed form, or GLP-1 (7-36) amide. Accordingly, the designation GLP-1 (7-37) designates that the fragment in question comprises the amino acid residues from (and including) number 7 to (and including) number 37 when counted from the N-terminal end of the parent peptide, GLP-1. The amino acid sequence of GLP-1(7-36)amide and of GLP-1(7-37) is given in formula I (SEQ ID No.: 43):

His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-X (I), which shows GLP-1(7-36)amide, when X is NH2, and GLP-1 (7-37), when X is Gly-OH.

GLP-1 is a gut hormone and is the most potent endogenous insulinotropic agent with actions that include stimulating adenylate cyclase and protein kinase activity in the beta-cell. Physiologically, together with gastric inhibitory polypeptide from the upper gut, it functions as an incretin hormone lowering the blood glucose level. Accordingly, GLP-1, secreted in response to food intake, has e.g. multiple effects on the stomach, liver, pancreas and brain that work in concert to regulate blood sugar. Conseqently, Glucagon-like peptide GLP-1(7-36) amide, and its non-amidated analogue GLP-1(7-37) have attracted considerable interest because of their potent actions on carbohydrate metabolism and its potential applicability to the treatment of diabetes, including type 2 diabetes.Type 2 diabetes is characterized by insulin resistance, since cells do not respond appropriately when insulin is present. This is a more complex problem than type 1 diabetes. Type 2 diabetes may go unnoticed for years in a patient before diagnosis, since the symptoms are typically milder (no ketoacidosis) and can be sporadic. However, severe complications can result from unnoticed type 2 diabetes, including renal failure, and coronary heart disease. This leads to increased morbidity and mortality.

GLP-1 (7-36) amide or GLP-1(7-37) is short-lived in serum. The peptide is cleaved by dipeptidyl peptidase IV (DPP-IV) between residues 8 and 9. The cleaved peptide is inactive. Thus GLP-1, administered exogenously, is extremely short-lived and has limited utility in therapeutic applications.

Various attempts have been made to synthesise stabilised (against DPP-IV) analogues of naturally occurring GLP-1 (GLP-1 (7-37)). In particular, the 8. residue, which in vivo is Ala, was replaced by another residue, for instance, Gly, Ser or Thr (Burcelin, R., et al. (1999) Metabolism 48, 252-258). The Gly8 or G8 analogue has been extensively tested, both as synthesised molecule, and produced by cell lines genetically engineered to secrete the mutant polypeptide (Burcelin, R., et a1 (1999) Annals of the New York Academy of Sciences 875: 277-285). Various other modifications have been introduced into GLP-1 (7-37) to enhance its in vivo stability without compromising its biological activity. However, all of these approaches did not achieve any significant therapeutic significance due to considerable problems involved.

In WO/9953064, Thorens, B. discloses a strategy for creating a multimeric GLP-1 expression cassette which can be incorporated into a variety of cell types which are publicly available immortalised cell lines and dividing primary cell cultures. Examples include EGF-responsive neurospheres, bFGF-responsive neural progenitor stem cells from the CNS of mammals, while the worked example uses baby hamster kidney, BHK cells. The implanted transfected cells were said to have been used to treat successfully diabetic mice, allowing glucose control equivalent substantially to non-diabetic controls. However, this techniques does not comply with the requirements of a treatment to be administered routinely to diabetes patients.

Another approach to stabilize the glucose level exogeneously is based on a new class of medicines known as incretin mimetics under investigation for the treatment of type 2 diabetes. Exenatide (Byetta®) is a synthetic version of a natural compound found in the saliva of the Gila monster lizard. In clinical trials, an incretin mimetic (exenatide) has demonstrated reductions in blood sugar and improvements in markers of beta cell function. However, Exenatide exhibits only certain effects of human incretin hormone glucagon-like peptide-1 (GLP-1).

In summary, at present there is no efficient diabetes type 2 therapy available, which allows to lower the blood glucose level on the basis of GLP-1, in other words to provide a therapy which reflects the entire spectrum of beneficial effects known for GLP-1, e.g. its activity in physiological concentrations to powerfully reduce the rate of entry of nutrients into the circulation by a reduction of gastric emptying rate in obese subjects or its insulin stimulating activity. Therefore, it is an object of the present invention to provide GLP-1 based peptide molecules which are biologically active and resistant towards proteolytic degradation.

The present invention relates to a fusion peptide comprising as component (I) N-terminally a GLP-1(7-35, 7-36 or 7-37) sequence and as component (II) C-terminally a peptide sequence of at least 9 amino acids or a functional fragment, variant or derivative thereof, whereby component (II) is characterized by a specific pattern of charged amino acids. Hereby, component (II) is a homologue of the naturally occurring IP2-sequence, which is coupled via its N-terminus (e.g. through a linker sequence or directly) to the C-terminus of component (II) which is a homologue of the native IP2 sequence. Homologue means a sequence derived from naturally occurring IP2 and sharing a certain sequence homology with IP2. In addition, the homologue exerts the same behaviour to component (I) as IP2 does, thereby enhancing e.g. the plasma stability of component (I). Typically, the homologue reflects a certain pattern of negatively charged amino acids as outlined by general formula (A).

The present invention is based on the finding that the resulting inventive peptide is protected against the proteolytic degradation in vivo, mainly due to proteolytic endopeptidase IV activity. The inventive peptide having at least two components (I) and (II) exhibits GLP-1's biologically activity and, simultaneously, confers stability to the GLP-1 as its component (I) by a C-terminal elongation.

The term "inventive peptide" as used herein is a fusion peptide as defined herein, a variant, an analog, a fragment or a derivative thereof, including combinations e.g. a derivatized fragment, analog or variant of a fusion peptide. The term "GLP-1 peptide" as used herein means GLP-1(7-35, 36 or 37), whereas "modified GLP-1 peptide" is intended to mean any GLP-1 analogue, a GLP-1 derivative, a GLP-1 variant or GLP-1 fragment, including a derivatized fragment, analog or variant of GLP-1(7-35, 36 or 37), which may occur in either component (I) and (III) of the inventive peptide. The term "GLP-2 peptide" as used herein means GLP-2 (1-33, 34, or 35), whereas "modified GLP-2 peptide" is intended to mean any GLP-2 analogue, fragment or variant, a GLP-2 derivative or a derivative of a GLP-2 analogue, including a derivatized fragment, analog or variant of GLP-2(1-33, 34 or 35). Variants, analogs, fragments and derivatives are categorized as modifications of the unmodified sequence, e.g. GLP-1(7-35, 36 or 37) or GLP-2(1-33, 34 or 35). Within the meaning of the present invention any variant, analog, fragment or derivative has to be functional, e.g. has to exert the same or a similar biological effects as the unmodified GLP-1 peptide.

Preferably, the inventive peptide is a fusion peptide or a variant, analog, fragment or derivative thereof, wherein component (I) contains a sequence having at least 80 %, more preferably at least 85 % and even more preferably at least 90 % sequence homology with SEQ ID No.: 1. SEQ ID No.1 represents the native amino acid sequence of GLP-1(7-37) (length of 31 amino acids), which is strictly conserved among mammalians.

The second component (component (II)) of the fusion peptide according to the invention (or more generally any inventive peptide including analogs, fragments, variants or derivatives of fusion peptides) typically contains a sequence length of at least nine amino acids, which may or may not form a β-turn like structure, however preferably forming a H-turn structure. A β-turn structure is a typical secondary structure element of proteins or peptides. It is typically formed by four amino acids, which revert the direction of the peptide's or protein's backbone chain direction. The amino acid sequence of component (II) contains at least nine amino acids and, preferably, at least two negatively charged residues (under physiological pH conditions). These negatively charged residues may preferably be apartate or glutamate or artifical amino acids not occurring in nature with carboxy function at their side chains. The at least two negatively charged residues may be located either in consecutive order (exhibiting e.g. the motifs: EE, DD, DE, ED, EED, EEE, EDE, DEE, DDD, DDE, DED, EDD, DDDD, EEEE, EDEE, EEDE, EEED, DDDE, DDEE etc.) or may be positioned anywhere within the C-terminal component (II) of the inventive fusion peptide. The N-terminal component of the fusion peptide may therefore be based on the general formula (A)

-NH₂-(X1_{b}-Y1ₐ-X2_{b}-Y2ₐ-X3_{b})_{c}-COOH (A)

whereby
Y1 and Y2 is a negatively charged amino acid as described above,
X1, X2 and X3 is any amino acid except for a negatively charged amino acid
a and b are from 0 to 15, whereby a may be identical or different for X1, X2, and X3, and
b may be different or identical for Y1 and Y2, and
c is from 1 to 5,
wherein,
if Y2 is E (glutamate) and a is 2 for Y2,
(i) a is not 0 or 2 for Y1, if Y1 is E, or (ii) X2 (for b >= 1) contains any amino acid apart from V, A or P (valin, alanin or prolin) as residue being neighboured to E of Y2, or (iii) X3 (for b >= 1) contains any amino acid apart from L or V (leucin or valin) as residue being neighboured to E of Y2, or (iv) c is not 1, if Y1 is E and a is 2 for Y1.

That is to say, the above formula (A) maintains its general character with the only exception given in case Y2 is E and a is 2 for Y2. In that case, the general formula is subjected to one of the following exceptions: (i) a is not 0 or 2 for Y1, if Y1 is E, or (ii) X2 (for b >= 1) contains any amino acid apart from V, A or P (alanin or prolin) as residue being neighboured to E of Y2, or (iii) X3 (for b >= 1) contains any amino acid apart from L or V (leucin or valin) as residue being neighboured to E of Y2, or (iv) c is not 1, if Y1 is E and a is 2 for Y1.
In other words, the general formula (A) is subject to one of those four exceptions given above, if Y2 is E (glutamate) and a is 2 for Y2. Exceptions (ii) and (iii) given above are meant to exclude embodiments, wherein the most C-terminal residue of X2 is neither V nor P nor A, or the the most N-terminal residue of X3 is neither L nor V. Specifically, if b (for X2 or X3) is 1, X2 or X3 cannot be V/P/A or L/V, respectively.

In a preferred embodiment, a is >=2 for Y2, if a=0 for Y1. Alternatively, if a = 1 for Y1, a for Y2 is typically >= 1. In a preferred embodiment, a for Y1 is = 0, 1, or 2, respectively, and a for Y2 is >= 2, preferably a for Y2 is 2, 3, or 4.

In a preferred embodiment of the present invention, component (II) of the inventive fusion peptide exhibits preferably more than 20% and, simultaneously, less than 40%, more preferred less than 50%, more preferred less than 60%, more preferred less than 70% und even more preferred less than 80% sequence homology to the native IP2 sequence. Even more preferred, component (II) does not correspond to the native sequence of IP2.

In another preferred embodiment, component (II) contains at least one posively charged residue (under physiological conditions). The positively charged residue may preferably be selected from arginin (R), lysine (K) or histidine (H) or may be any other (non-native) amino acid with a positively charged side chain function. It is further preferred for component (II) to encompass 2 to 4 positively charged residues. Irrespective of the number of positively charged residues, they may be located at any position within the component (II). In case more than one positively charged amino acid occurs within component (II), they may be in consecutive order or located at various positions within component (II). With regard to the above formula (A), it is preferred that X1, X2 and/or X3 are R, K or H, if b is 1 or, if b is > 1, it is preferred that the stretches of the amino acids formed by X1b (b>1), X2b (b>1) and/or X3b (b>1) stretches contain at least 1, preferably more than 1 positively charged residue.

In another preferred embodiment, component (II) contains at least one proline or alanine residue in its sequence. Proline residues are common amino acids within the β turn forming tetrameric amino acid sequence. The proline residue (P) is commonly is typically located at position 2 or 3, preferably 2, of the tetrameric β-turn sequence occurring in component (II) of the fusion peptide. That is to say, if b is at 4 or >4, amino acid stretches formed by X1 or X2 may preferably contain at least be P residue.

Preferred amino acid motifs, which may be contained in component (II) and are covered by general formula (A), are DFP(EE)A, DFPEEI, DFP(EE)L, DFPEET, DFP(EE)S, DFPEEG, P(EE)SAI, P(EE)TAI, P(EE)LAI, PEEIAI, P(EE)AAI, PEEGAI, G(EE)VAI, TEEVAI, A(EE)VAI, LEEVAI, S(EE)VAI, MEEVAI, F(EE)VAI, IAEEA, IA(EE)T, IAEES, IA(EE)G, IAEEP, IA(EE)V, IAEEM, IA(EE)W, IA(EE)Y, or, IA(EE)F. All of the afore-mentioned motifs may exhibit, instead of the (EE) core, the variants given above, e.g. (DD), (DE), (ED), (EED), (EEE), (EDE), (DEE), (DDD), (DDE), (DED), (EDD), (DDDD), (EEEE), (EDEE), (EEDE), (EEED), (DDDE), (DDEE) etc.. These motifs may be located anywhere in the sequence of component (II).

An inventive fusion may typically have in its component (II) sequence length of 9 to 30, preferably 9 to 20, and most preferably 9 to 15 amino acids. Generally spoken, shorter sequences in component (II) may be preferred due to their superior binding activity to the GLP receptor over longer sequences. The sequence of component (II), even though it is not a prerequisite, may preferably have a net negative charge at pH 7 due to the occurrence of at least two negatively charged residues or may have neutral net charge due the occurrence of additional positively charged residues as outlined above.

The inventive fusion peptide component (II) is a peptide sequence being linked to the C-terminus of component (I). It is preferred, if the very N-terminal residues of component (II), which are linked to the C-terminus of component (I), exhibit a sequence motif selected from the group consisting of AA, XA, AX, RR, RX, XR, KK, KX, and XK, wherein X represents any amino acid (naturally occurring or a modified non-natural amino acid). Alternatively, the very N-terminal residues of component (II) may as well be selected from the group consisting of EE, EX, XE, DE, ED, DD, XD, DX, wherein X represents again any amino acid (naturally occurring or a modified non-natural amino acid).

A preferred motif occurring in component (II) of an inventive fusion peptide contains a sequence having more than 20%, more preferred more than 30%, more preferred more than 40% and even more preferred more than 50%, and, simultaneously, less than 80% sequence homology to the sequence motif SEQ ID No.: 25 (DFPEEVA), whereby typically the amino acid P and/or the amino acid V of that sequence motif are substituted by any other amino acid sequence. Particularly preferred is a fusion peptide, wherein component (II) contains a sequence having more than 20%, more preferred more than 30%, more preferred more than 40% and even more preferred more than 50%, and, simultaneously, less than 85%, preferably less than 80% sequence homology to the sequence according to SEQ ID No.: 22 (RRDFPEEVAI) or SEQ ID No.: 26 (AADFPEEVAI), which contains a partial sequence of the full-length native (human or murine) IP-2 (intervening peptide 2) sequence. Hereby, typically the amino acid P and/or the amino acid V of sequence ID No. 22 or SEQ ID No. 26 are substituted by any other amino acid. SEQ ID No.: 26 and SEQ ID No.: 22 are distinct by substitution of the N-terminal (RR) residues by (AA). Stronger preferred sequences being contained in component (II) are derivatives of longer partial amino acid sequences of IP-2, such as sequences having more than 20%, more preferred more than 30%, more preferred more than 40% and even more preferred more than 50%, and, simultaneously, less than 95 or less than 90%, more preferably less than 85%, even more preferably less than 80% sequence homology to SEQ ID No.: 23 (RRDFPEEVAIVEEL)) or SEQ ID No. 24 (RRDFPEEVAIAEEL)). Hereby, typically the amino acid A (at position 11 of SEQ. ID No. 24), P, L and/or the amino acid V (at position 8 of both sequences) of sequence ID No. 23 or SEQ ID No. 24 are substituted by any other amino acid. Most preferred as elements being contained in component (II) of the fusion peptide are sequences having more than 20%, more preferred more than 30%, more preferred more than 40% and even more preferred more than 50%, and, simultaneously, less than 95% or less than 90%, more preferably less than 85%, even more preferably less than 80% sequence homology SEQ ID No.: 2 (RRDFPEEVAIVEELG) or SEQ ID No. 3 (RRDFPEEVAIAEELG). Again, hereby the amino acid A (at position 11 of SEQ. ID No. 3), P, L and/or the amino acid V of sequence ID No. 2 or SEQ ID No. 3 are typically substituted by any other amino acid sequence. The sequences contained in component (II) of the inventive fusion peptide may also be derived from SEQ ID No.: 27 (AADFPEEVAIVEEL), SEQ ID No.: 28 (AADFPEEVAIAEEL), SEQ ID No.: 29 (AADFPEEVAIVEELG), and SEQ ID NO.: 30 (AADFPEEVAIAEELG) by provision of homologs thereof having at least 20%, more preferred more than 30%, more preferred more than 40% and even more preferred more than 50%, and, simultaneously, less than 95% or less than 90%, more preferably less than 85%, even more preferably less than 80% sequence homology. Hereby, component (II) contains typically sequences with the substitutions disclosed in this paragraph.

Component (II) may contain more than one copy of a homologue as described in this paragraph, e.g. 2, 3 or even more copies.

The above homologues are preferably substitution derivatives of SEQ ID No. 2,3, 22, 23, 24, 26, 27, 28, 29, 30, that is to say the modifcations introduced correspond to substitutions.

Moreover, an inventive peptide is preferred containing a homologue derived from a parent sequence selected from the group consisting of SEQ ID No.: 8 (HAEGTFTSDVSSYLEGQAAKEFIAWLVKGRGRRDFPEEVAIAEELG), according to SEQ ID No.: 12 (HAEGTFTSDVSSYLEGQAAKEFIAWLVKGRGRRDFPEEVAIVEELG), SEQ ID No.: 31 (HAEGTFTSDVSSYLEGQAAKEFIAWLVKGRGAADFPEEVAIAEELG), SEQ ID No.: 32 (HAEGTFTSDVSSYLEGQAAKEFIAWLVKGRGRRDFAEEVAIAEELG), SEQ ID No.: 33 (HAEGTFTSDVSSYLEGQAAKEFIAWLVKGRGRRDAAAAVAIAEELG), SEQ ID No.: 34 (HAEGTFTSDVSSYLEGQAAKEFIAWLVKGRGAADAAAAVAIAAALG), SEQ ID No.: 35 (HAEGTFTSDVSSYLEGQAAKEFIAWLVKGRGRRDFP), SEQ ID No.: 36 (HAEGTFTSDVSSYLEGQAAKEFIAWLVKGRGRRDFPEEVA), SEQ ID No.: 37 (HAEGTFTSDVSSYLEGQAAKEFIAWLVKGRGRRDFPEEVAIAEELGRRHAC), SEQ ID No.: 38 (HAEGTFTSDVSSYLEGQAAKEFIAWLVKGRGAADFPEEVAIVEELG), SEQ ID No.: 39 (HAEGTFTSDVSSYLEGQAAKEFIAWLVKGRGRRDFAEEVAIVEELG), SEQ ID No.: 40 (HAEGTFTSDVSSYLEGQAAKEFIAWLVKGRGRRDAAAAVAIVEELG), SEQ ID No.: 41 (HAEGTFTSDVSSYLEGQAAKEFIAWLVKGRGAADAAAAVAIVAALG), SEQ ID No.: 42 (HAEGTFTSDVSSYLEGQAAKEFIAWLVKGRGRRDFPEEVAIVEELGRRHAC), whereby the homolgue has preferably less than 95% or less than 90%, more preferably less than 85%, even more preferably less than 80% sequence homology and, simultaneously, more than 20%, more preferred more than 30%, more preferred more than 40% and even more preferred more than 50% sequence homology to the above parent sequence. Typical inventive derivatives of the above parent sequences exhibit specific modification patterns, whereby those of the above sequences exhibiting the motif (AEEL) and/or (PEEV) have at least one modification (substitution) at any one position of either one or both of these motif(s). Accordingly, the above homologues may have a substitution as disclosed herein, e.g. exchange of the amino acids A, L, P or V by any other amino acid or exchange of one or both of E residues by a D residue. In all of these embodiments, GLP-1(7-37) is linked without any linker sequence via its C-terminus to component (II) which is a homologue of native IP2 sequence.

Without being bound to any theory, it is concluded by the inventors of the present invention that the instability of GLP-1(7-35, 36 or 37), if administered to any patient in need thereof, is due to its unprotected 3-dimensional structure. Proteases may cleave the GLP-1(7-35, 36 or 37) peptide and abolish its physiological activity rapidly in vivo. By linking a peptide sequence to the C-Terminus of GLP-1(7-35, 36 or 37) its structure gains stability towards enzymatic degradation. Gain in stability appears to be enhanced, if the additional C-terminal peptide sequence (being contained in component (II) of the fusion peptide according to the invention) folds back due to the presence of a r1-turn structural element formed by its primary structure and providing rigidity to component (II). However, a f3-turn structure in component (II) of the inventive peptide does not appear to be a prerequisite for stabilizing the GLP-1 sequence of component (I) towards enzymatic degradation. Accordingly, component (II) of the inventive fusion peptide may also trigger structural modifications within component (I), thereby protecting the inventive fusion peptide against enzymatic degradation. The inventive peptide, by virtue of its C-terminal peptide extension, e.g. containing a β-turn structural element, is found to have improved resistance to DPP-IV inactivation. The C-terminal peptide is either not cleaved from the GLP-1 (7-35, 36 or 37) sequence prior to acting on its receptor in target cells or it may be cleaved enzymatically to form GLP-1 (7-35, 36 or 37) in vivo. Irrespective of the exact form of the inventive peptide bound at the site of the GLP-1 receptor, an inventive peptide exerts its function as an active insulinotropic compound.

Peptide sequences, which are considered to be suitable for being contained in component (II) due to a primary structure forming a r1-turn element may readily be identified by adequate e.g. spectroscopic methods, e.g. circular dichroism, or other methods known to the skilled person.

Component (II) and component (I) may be directly linked or linked via a linker sequence. Preferably, both components are directly linked with each other. In case they are linked via a linker (or spacer), the linker is preferably a peptide linker or an organic linker. A peptide linker typically has a length of 1 to 10 amino acids, preferably 1 to 5, even more preferably 1 to 3 amino acids, in some cases the linker sequence may be even longer comprising 11 to 50 amino acids. A peptide linker may be composed of various amino acid sequences. Preferably, a peptide linker will introduce some structural flexibility between components to be linked. Structural flexibility is achieved e.g. by having a peptide linker containing various glycine or proline residues, preferably at least 30%, more preferably at least 40% and even more preferably at least 60 % proline and glycine residues within the linker sequence. Irrespective of the specific sequence the peptide linker may preferably be immunologically inactive.

In a preferred embodiment of the present invention, an inventive peptide, i.e. a fusion peptide as described above, contains a third component (component (III)) which is either linked to the C-terminus of component (II) and/or to the N-terminus of component (I). Preferably, component (III) is located at the C-terminus of component (II). Irrespective of whether component (III) is linked to N-terminus of component (I) (by its C-terminus) or to the C-terminus of component (II) (by its N-terminus), the coupling may be direct or indirect via a linker sequence. With regard to the linker sequence it is referred to the above disclosure for a linker connecting component (I) and component (II). Generally, component (III) comprises at least four amino acid residues, preferably at least 10 additional amino acid residues, more preferably at least 20, or at least 30. In functional terms, component (III) is provided to further enhance the stability of an inventive peptide. Component (III) is expected not to interfere with the biological function of the inventive peptide which is app. comparable to the biological activity of GLP-1(7-37).

Preferably, component (III) of the inventive peptide comprises at least 4, preferably at least 10, more preferably at least 20 additional amino acid residues of the N-terminal sequence of an isoform of GLP-2 of any mammalian organism (other naturally occurring variant of GLP-2 among mammalian), e.g. murine or human isoforms as shown in SEQ ID Nos: 4 and 5. GLP-2 occurs in pro-glucagon and is also involved in carbohydrate metabolism. As with the biologically active sequence included in component (I) (GLP-1 peptide), component (III) may also comprise analogs, variants or derivatives of naturally occurring forms of GLP-2. Alternatively, component (III) may also comprise at least 4, preferably at least 10, more preferably at least 20 additional amino acid residues of the N-terminal sequence of GLP-1(7-37), correspondingly including all mammalian isoforms or - as disclosed herein - all functional variants, analogs or derivatives thereof. Generally speaking, component (III) may contain any form of a GLP-1 peptide or a modified GLP-1 peptide, which is disclosed herein as suitable for component (I) of the inventive peptide. In a further alternative, component (III) may also contain chimeric forms of GLP-1(7-37) and GLP-2. A chimeric form may be produced by coupling GLP-1 (7-37) and GLP-2 (or fragments, analogs, variants or derivatives of both) with each other and by subsequently introducing this chimeric form as component (III) into the inventive peptide. Preferably, the chimeric form is composed of a partial sequence of GLP-1(7-37) and a partial sequence of GLP-2 linked together. E.g. the chimeric form may include the N-terminal 5 to 30 amino acids of GLP-1 and the C-terminal 5 to 30 amino acids of GLP-2 or vice versa, e.g amino acids 7 or 8 to 22, 23, 24, 25, 26, 27, or 28 of GLP-1(7-37) and amino acid sequence from position 15, 16, 17, 18, 19, 20, 21, 22, 23 or 24 to e.g. the C-terminus of GLP-2.

If modifications of naturally occurring forms of GLP-2 or GLP-1(7-37), respectively, are used as component (III), component (III) preferably contains the sequence of SEQ ID Nos.: 4 or 5 or SEQ ID No.: 1, respectively, or a sequence having at least 80% sequence homology with SEQ ID Nos.: 4 or 5 or SEQ ID No.: 1. Derivatives of these preferred sequences, e.g. due to side chain modifications or peptide backbone modifications etc. (as disclosed herein pertaining to "derivatives"), are also encompassed as component (III) by the present invention.

In another embodiment, component (III) may contain a plurality of sequences as described above. E.g. component (III) may contain at least two, preferably 2, 3, or 4 copies of GLP-1(7-37) and/or GLP-2 or at least two copies of sequences having at least 80% sequence homology with SEQ ID Nos: 1, 4 or 5. Also component (III) may contain more than copy of a chimeric version of GLP-1 (7-37) or GLP-2, as disclosed above, e.g. eventually forming a combination of chimeric version(s) together with GLP-1(7-37) and/or GLP-2 or its modifications with at least 80 % sequence homology. Within the scope of the present invention are also two or more, preferably two component (III), which may e.g. be (1) linked by its N-terminus to the C-terminus of component (II) and (2) linked by its C-terminus to the N-terminus of component (I) via a linker or directly. If two components (III) are provided, these may be identical or different.

Accordingly, inventive fusion peptides containing three components (I), (II) and (III) are particularly preferred. Four specific embodiments containing all of these components are selected from a group consisting of homologues derived from the following SEQ ID Nos.: SEQ ID No. 6 (N-GLP-1(7-37)--RR-GLP-1(7-37)-C, also designated murine CM1 herein), SEQ ID No. 7 (N-GLP-1(7-37)-IP2(murine)-RR-GLP2-C, also designated murine CM2 herein), SEQ ID No. 10 (N-GLP-1(7-37)-IP2(human)-RR-GLP-1(7-37)-C, also designated human CM1), and SEQ ID No. 11 (N-GLP-1(7-37)-IP2(human)-RR-GLP-2-C), also designated human CM2 herein), whereby the homologues are characterized by homologues (component (II)) of the native IP2 sequences as given for the afore-mentioned SEQ ID Nos. With regard to the homologous character (IP2 homologues) it is referred to the disclosure herein for component (II). Component (I) in each of the embodiments according to SEQ ID Nos: 6, 7, 10 or 11 is GLP-1(7-37), whereas component (III) (in each of these embodiments linked to the C-terminus of component (II)) is either GLP-1(7-37) or GLP-2. An RR linker links the IP2 homologue (component (II)) to component (III).

In a preferred embodiment, the inventive fusion peptide contains at least one polymer constituent which is typically covalently coupled to the fusion peptide subunit. "Conjugated" in the meaning of the present invention is intended to mean "chemically coupled". "Chemically coupled" is intended to mean coupled via covalent or non-covalent bondings. While covalent bonding is preferred, the polymer constituent may also be linked to the fusion peptide via complexation without covalent linkage, e.g. via hydrogen bonding or electrostatic, hydrophobic, etc. interaction. The entire complex containing the fusion peptide and the polymer is termed hereinafter "conjugate complex" or "conjugate molecule".

The conjugate complex having an inventive peptide of at least two components (I) and (II) and the synthetic polymer exhibits GLP-1's biologically, activity and, simultaneously, confers stability to the GLP-1 as its component (I) by a C-terminal elongation. Accordingly, by conjugating the fusion peptide to a polymer its in vivo stabilisation is increased considerably.

The polymer used herein can be a physiologically acceptable polymer which includes polymers which are soluble in an aqueous solution or suspension and have no negative impact, such as side effects, to mammals upon administration of the fusion peptide in a pharmaceutically effective amount. There is no particular limitation to the physiologically acceptable polymer used according to the present invention. The polymer may be of synthetic nature or may be naturally occurring polymer (natural polymer, e.g. a protein). Specific embodiments of polymers are disclosed hereinafter.

One, two, or three polymer constituents may be covalently attached to the fusion peptide subunit, with one polymer constituent being preferred, to form the inventive conjugate molecule. However, in specific embodiments more than three polymer constituents may be provided per fusion peptide subunit. The constituents may be covalently coupled to either component (I) or component (II) of the fusion peptide or both of them. It is preferred to couple at least one of the polymer constituents to component (II). If one or more polymer constituent(s) is/are coupled to component (I) it/they is/are preferred to be coupled to the N-terminus or to the side chains of serine, threonine, tyrosine, aspartate, glutamate, lysine or arginine residues. Preferably, the side chains of one or more of residues Thr 11, Thr 13, Asp15, Ser 17, Ser 18, Tyr 19, Glu 21, Lys 26, Glu 27, Lys 34, Arg 36 of component (I) are used for coupling purposes. If the naturally occurring sequence of IP2 is used as component (II) one or more of its Arg, Glu and Asp residues will modified at their side chains by a polymer constituent(s).

The N-terminal binding of a polymer, in particular PEG, may offer advantages in purification of the conjugate. It is also believed that the N-terminal binding of a polymer may better preserve the bioactivity compared with random polymer binding to any other residue, e.g. any other lysine residue. Accordingly, in a preferred embodiment, at least one polymer constituent is located at the N-terminus of the fusion peptide. If PEGylation is used, PEGylation of terminal or side chain carboxyl groups or the epsilon-amino group of lysine occurring in the inventive peptide, confers resistance to oxidation and is also within the scope of the present invention.

Polymers used in the fusion peptide according to the present invention can be prepared by polymerising in the usual way monomers of the following formula: 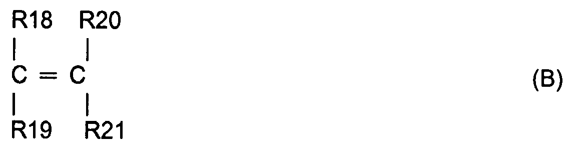 in which
R18 is selected from hydrogen, halogen, C1-4 alkyl and groups COOR22 in which R22 is hydrogen and C1-4 alkyl;
R19 is selected from hydrogen, halogen and C1-4 alkyl;
R20 is selected from hydrogen, halogen, C1-4 alkyl and groups COOR22 provided that R18 and R20 are not both COOR22; and
R21 is a C1-10 alkyl, a C1-20 alkoxycarbonyl, a mono- or di-(C1-20 alkyl) amino carbonyl, a C6-20 aryl (including alkaryl), a C7-20 aralkyl, a C6-20 aryloxycarbonyl, a C1-20 aralkyloxycarbonyl, a C6-20 arylamino carbonyl, a C7-20 aralkyl-amino, a hydroxyl or a C2-10 acyloxy group, any of which may have one or more substituents selected from halogen atoms, alkoxy, oligo-alkoxy, aryloxy, acyloxy, aclamino, amine (including mono and di-alkyl amino and trialkylammonium), carboxyl, sulphonyl, phosphoryl, phosphino, (including mono- and di-alkyl phosphine and tri-alkylphosphonium), zwitterionic and hydroxyl groups;
or R21 and R20 or R21 and R19 may together form -CONR23CO in which R23 is a C1-20 alkyl group.

The ethylenically unsaturated monomers may polymerise to form a polymer selected from polyalkylene glycol, polyvinylpyrrolidone, polyacrylate, poloyoxazoline, polyvinylalcohol, polacrylamid, polymethacrylmide or a HPMA copolymer. Alternatively, the polymer may be susceptible to enzymatic or hydrolytic degradation, such as polyesters, polyacetales, poly(orthoesters), polycarbonates and polyamides.

The polymer may be a homopolymer or a copolymer of at least two of the above mentioned components. The copolymer can be a block copolymer or a random copolymer.

Specific particularily preferred examples of the polymers include, but are not limited to, poly alkylene glycols such as polyethylene glycol (PEG), polypropylene glycol (PPG), copolymers of ethylene glycol and propylene glycol and the like, polyoxyethylated polyol, polyolefinic alcohol, polyvinylpyrrolidone, polyhydroxyalkyl methacrylamide, polyhydroxyalkyl methacrylate, such as polyhydroxyethylen methycrylate, polyacrylate, polysaccharides, poly([alpha]-hydroxy acid), polyvinyl alcohol, polyphosphazene, polyoxazoline, poly(N-acryloylmorpholine), polyvinylethyl ether, polyvinlyacetale, polylactic glycolic acid, polylactic acid , lipid polymer, chitin, hyaluronuic acid, polyurethyne, polysialic acid cellulose triacetat, cellulose nitrate and combinations of any of the foregoing.

Further specific polymers may be selected from N-(2-hydroxypropyl)methacrylamide (HPMA), and poly(lactide-co-glycolide) (PLGA) copolymers, poly(N-vinylpyrrolidone-co-acrylic acid-g-PEG) (VAP), a copolymer of N-vinylpyrrolidone with acrylic acid, and a copolymer of N-vinylpyrrolidone with maleic anhydride.

The inventive peptides may occur in various modified forms. These modified forms are disclosed in the following and described in more detail.

The term "salts" herein refers to both salts of carboxyl groups and to acid addition salts of amino groups of the fusion peptides described above or analogs, fragments, derivatives or variants thereof. Salts of a carboxyl group may be formed by means known in the art and include inorganic salts, for example, sodium, calcium, ammonium, ferric or zinc salts, and the like, and salts with organic bases as those formed, for example, with amines, such ethanolamine, arginine or lysine, piperidine, procaine and the like. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of the inventive peptides relevant to the present invention, i.e. the ability to reduce the rate of entry of nutrients into the circulation. As disclosed below, the salt peptide forms may be contained in a pharmaceutical formulation.

A "fragment" of a fusion peptide according to the present invention refers to any subset of the molecules, that is, a shorter peptide which retains the desired biological activity. Fragments may readily be prepared by removing amino acids from either end of the molecule and testing the resultant for its properties as a incretin. Proteases for removing one amino acid at a time from either the N-terminal and/or the C-terminal of a polypeptide are known, and so determining fragments which retain the desired biological activity involves only routine experimentation. Conclusively, fragments may be due to deletions of amino acids at the peptide termini and/or of amino acids positioned within the peptide sequence.

Additionally, the inventive peptide which has anti-diabetes type 2 activity, be it a fusion peptide itself, an analog or variant, salt, functional derivative and/or fragment thereof, can also contain additional amino acid residues flanking the inventive peptide. As long as the resultant molecule retains its resistancy or stability towards proteases and its ability to act as incretin, one can determine whether any such flanking residues affect the basic and novel characteristics of the core peptide, e.g. by its effects on pancreas cells, by routine experimentation. The term "consisting essentially of, when referring to a specified sequence, means that additional flanking residues can be present which do not affect the basic and novel characteristic of the specified inventive peptide. This term does not comprehend substitutions, deletions or additions within the specified sequence.

A "variant" according to the present invention refers to a molecule which is substantially similar to either the entire inventive peptide defined above or a fragment thereof. Variant peptides may be conveniently prepared by direct chemical synthesis of the variant peptide, using methods well known in the art. Of course, such variant of an inventive peptide would have similar anti-diabetic, e.g. insulin stimulating activity as the corresponding naturally-occurring GLP-1 peptide.

Alternatively, amino acid sequence variants of the peptides defined above can be prepared by mutations in the DNAs which encode the synthesized derivatives. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure.

An "analog" of the peptides defined above, according to the present invention, refers to a non-natural molecule which is substantially similar to either the entire molecule or to an active fragment thereof. Such analog would exhibit the same activity as the corresponding naturally-occurring GLP-1 peptide.

The types of substitutions which may be made in the inventive peptide, according to the present invention, may be based on analysis of the frequencies of amino acid changes between a homologous protein/peptide of different species. Based upon such analysis, conservative substitutions may be defined herein as exchanges within one of the following five groups:
I. Small, aliphatic, non-polar or slightly polar residues: Ala, Ser, Thr, Pro, Gly; II. Polar, negatively-charged residues and their amides: Asp, Asn, Glu, Gin; III. Polar, positively-charged residues: His, Arg, Lys; IV. Large, aliphatic non-polar residues: Met, Leu, Ile, Val, Cys ; V. Large aromatic residues: Phe, Try, Trp.

Within the foregoing groups, the following substitutions are considered to be "highly conservative" : Asp/Glu; His/Arg/Lys; Phe/Tyr/Trp; Met/Leu/Ile/Val. Semi-conservative substitutions are defined to be exchanges between two of groups (I) - (IV) above which are limited to supergroup (A), comprising(I), (II), and(III) above, or to supergroup (B), comprising (IV) and (V) above. Substitutions are not limited to the genetically encoded or even the naturally-occurring amino acids.

In general, analogs or variants of the inventive peptide may also contain amino acid substitutions, made e.g. with the intention of improving solubility (replacement of hydrophobic amino acids with hydrophilic amino acids). In one embodiment of variants/analogs of the GLP-1 peptide of the inventive peptide (occurring in component (I) and/or (III) of the inventive peptide) the (modified) GLP-1 peptide is characterized by one or more substitution(s) at positions 7, 8, 11, 12, 16, 22, 23, 24, 25, 27, 30, 33, 34, 35, 36, or 37 of the GLP-1 peptide. As an example for the following nomenclature [Arg34-GLP-1 (7-37)] designates a GLP-1 analogue wherein the naturally occurring lysine at position 34 has been substituted with arginine.

Specifically, component (I) and/or (III) of an inventive peptide may comprise variants and analogs of GLP-1(7-35, 36 or 37) including, for example, Gln9-GLP-1 (7-37), D-Gln9-GLP-1(7-37), acetyl-Lys9-GLP-1 (7-37), Thr16-Lys18-GLP-1 (7-37), and Lys18-GLP-1 (7-37), Arg34-GLP-1 (7-37), Lys38-Arg26-GLP-1 (7-38)-OH, Lys36-Arg26-GLP-1 (7-36), Arg26,34-Lys38-GLP-1 (7-38), Arg26,34-Lys38-GLP-1(7-38), Arg26,34-Lys38-GLP-1 (7-38), Arg26,34-Lys38-GLP-1 (7-38), Arg26,34-Lys38-GLP-1 (7-38), Arg26-Lys38-GLP-1(7-38), Arg26-Lys38-GLP-1(7-38), Arg26-Lys38-GLP-1 (7-38), Arg34-Lys38-GLP-1 (7-38), Ala37-Lys38-GLP-1 (7-38), and Lys37-GLP-1 (7-37).

In another embodiment of the invention the inventive peptide contains as component (I) or (III) a modified GLP-1 peptide comprising the amino acid sequence of the following formula II (SEQ ID No.: 44):
Xaa7 -Xaa8-Glu-Gly- Thr-Phe- Thr-Ser-Asp-Xaa 16-Ser-Xaa18-Xaa 19-Xaa20-Glu-Xaa22-Xaa23-Ala-Xaa25-Xaa26-Xaa27-Phe-I le-Xaa3o-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is is Ser, Lys or Arg; Xaa19 is Tyr or Gln ; Xaa20 is Leu or Met; Xaa22 is Gly, Glu or Aib; Xaa23 is Gln, Glu, Lys or Arg ; Xaa25 is Ala or Val ; Xaa26 is Lys, Glu or Arg; Xaa27 is Glu or Leu; Xaa30 isAla, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent.

In another embodiment of the invention component (I) and/or (III) of the inventive peptide contains a modified GLP-1 peptide comprising the amino acid sequence of the following formula III (SEQ ID No.: 45):
Xaa7-XaaB-Glu-Gly-Thr-Phe-Thr-Ser-Asp-Val-Ser-XaaB-Tyr-Leu-Glu-Xaa22-Xaa23-Ala-Ala-Xaa26-Glu-Phe-Ile-Xaa30-Trp-Leu-Val-Xaa34-Xaa35-Xaa36-Xaa37,
wherein Xaa7 is L-histidine, D-histidine, desamino-histidine, 2-amino-histidine, -hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg ; Xaa26 is Lys, Glu or Arg;Xaa30 isAla, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent.

In a particular preferred embodiment of the invention component (I) and/or (III) of the inventive peptide contain a (modified) GLP-1 peptide, which is selected from GLP-1 (7-35), GLP-1 (7-36), GLP-1 (7-36)-amide, GLP-1 (7-37) or an variant, analogue or derivative thereof. Also preferred are inventive peptides comprising in their components (I) and/or (III) a modified GLP-1 peptide having a Aib residue in position 8 or an amino acid residue in position 7 of said GLP-1 peptide, which is selected from the group consisting of D-histidine, desamino-histidine, 2-amino-histidine, hydroxy-histidine, homohistidine, N-acetyl-histidine, a-fluoromethyl-histidine, a-methyl-histidine, 3-pyridylalanine, 2-pyridylalanine and 4-pyridylalanine.

Examples of production of amino acid substitutions in proteins which can be used for obtaining analogs for use in the present invention include any known method steps, such as presented in U. S. patents RE 33,653; 4,959,314; 4,588,585 and 4,737,462, to Mark et al; 5,116,943 to Koths et al; 4,965,195 to Namen et al; and 5,017,691 to Lee, et al, and lysine substituted proteins presented in US patent 4,904,584 (Shaw et al).

Preferably, the variant or analog, as defined above and contained in component (I) and/or (III), will have a core sequence, which is the same as that of the "native" sequence, e.g. GLP-1(7-37) or GLP-2 or biologically active fragment thereof with typically 70%, more preferably 80% amd even more preferably 90% sequence homology to the native precursor sequence. Component (II) is a IP2 homologue, which has an amino acid sequence having typically less than 95%, more preferably less than 90% and even more preferably less than 80% homology to native IP2 and/or corresponds to general formula (A). The variant or analog retains the biological activity of native GLP-1 thereof. More preferably, the entire fusion peptide (be it composed of component (I), (II) and (III) or of component (I) and (II)) will have a sequence of at least 80% identity, at least 90% identity, or most preferably at least 95% identity to the native pürecursor sequence. Where a particular peptide is said to have a specific percent identity to a reference polypeptide of a defined length, the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Of course, other polypeptides will meet the same criteria. The term "sequence identity" as used herein means that the sequences are compared as follows. The sequences are aligned using Version 9 of the Genetic Computing Group's GAP (global alignment program), using the default (BLO-SUM62) matrix (values-4 to +11) with a gap open penalty of-12 (for the first null of a gap) and a gap extension penalty of-4 (per each additional consecutive null in the gap). After alignment, percentage identity is calculated by expressing the number of matches as a percentage of the number of amino acids in the claimed sequence.

Derivatives of a fusion peptide or an analog, fragment or variant thereof are also encompassed by the present invention. The term "derivatives" of an inventive peptide is intended to include only those modified inventive peptides that do not change one amino acid to another of the twenty commonly-occurring natural amino acids. Correspondingly, a genetically encoded amino acid may be modified by reacting it with an organic derivatizing agent that is capable of reacting with selected side chains of residues or amino or carboxy groups of terminal residues (preferably by covalent modification) or by introducing non-natural amino acids (manufactured by chemical synthesis, i.e. D-isomers of the amino acids encoded by the genetic code, Aib (a-aminoisobutyric acid), Abu (a-aminobutyric acid), Tie (tert-butylglycine), p-aianine, 3-aminomethyl benzoic acid, anthranilic acid) or natural amino acids which are not encoded by the genetic code, e.g. hydroxyproline, γ-carboxyglutamate, ornithine, phosphoserine, D-alanine and D-glutamine or by a modification of the peptide backbone by alternative peptide backbone arrangements.

In the following preferred modifications of amino acids of the inventive peptide (which - as defined above - also comprises variants, analogues or fragments of the fusion peptide) are disclosed, which may occur in an inventive peptide at any site (any amino acid), e.g. positioned in component (I), (II) and/or (III).

Cysteinyl residues, if present in any form of an inventive peptide, e.g. an analogue of an inventive peptide, most commonly are reacted with alpha-haloacetates (and corresponding amines), such as chloroacetic acid or chloroacetamide, to give carboxylmethyl orcarboxyamidomethyl derivatives. Cysteinyl residues also are derivatized by reaction with bromotrifluoroacetone, alpha-bromo-beta-(5-imidazoyl)propionic acid, chloroacetyl phosphate, N-alkylmaleimides, 3-nitro-2-pyridyl disulfide, methyl-2-pyridyl disulfide, p-chloromercuribenzoate, 2-chloromercuri-4-nitrophenol, orchloro-7-nitrobenzo-2-oxa-1,3-diazole.

Histidyl residues in an inventive peptide may be derivatized by reaction with diethylprocarbonate at pH 5.5-7.0 because this agent is relatively specific for the histidyl side chain. Parabromophenacyl bromide is also useful; the reaction is preferably performed in 0.1 M sodium cacodylate at pH 6.0. In particular, the N-terminal histidine residue (His7) of GLP-1(7-37) as contained in component (I) and/or (III) of the inventive peptide is very important to the insulinotropic activity of GLP-1 peptides as shown by Suzuki et. al. (Diabetes Res.; Clinical Practice 5 (Supp. 1): S30 (1988)). Correspondingly, the inventive peptide may be modified at His7 of its GLP-1 as part of component (I) and/or (III) by alkyl or acyl (C1-C6) groups, or replacement of His with functionally-equivalent C5-C6 ring structures. A preferred modification is the introduction of a hydrophobic moiety at the amino terminus of His7 or its histidyl side chain.

Lysinyl and amino terminal residues of an inventive peptide may e.g. be reacted with succinic or other carboxylic acid anhydrides. Derivatization with these agents has the effect of reversing the charge of the lysinyl residues. By acyl (C12-C18) modifications of the epsilon-amino group of lysine residue(s) in the inventive peptide, their half-life in circulation is increased. Arginyl residues may e.g. be modified by reaction with one or several conventional reagents, among them phenylglyoxal; and ninhydrin. Derivatization of arginine residues requires that the reaction be performed in alkaline conditions because of the high pKa of the guanidine functional group. Furthermore, these reagents may react with the groups of lysine, as well as the arginine epsilon-amino group.

The specific modification of tyrosyl residues per se has been studied extensively. Most commonly, N-acetylimidazole and tetranitromethane may be used to form O-acetyl tyrosyl species and e-nitro derivatives, respectively.

Carboxyl side groups (aspartyl or glutamyl) may be selectively modified by reaction with carbodiimides (R'N-C-N-R') such as 1-cyclohexyl-3-[2-morpholinyl-(4-ethyl)] carbodiimide or 1-ethyl-3-(4-azonia-4,4-dimethylpentyl)carbodiimide.

Furthermore, aspartyl and glutamyl residues may be converted to asparaginyl and glutaminyl residues by reaction with ammonium ions.

Glutaminyl and asparaginyl residues may be deamidated to the corresponding glutamyl and aspartyl residues. Alternatively, these residues may be deamidated under mildly acidic conditions. Either form of these residues falls within the scope of this invention. Deamidated inventive peptides may undergo an altered susceptibility to proteolysis with protease or peptidase enzymes, suggesting that deamidation may have physiological significance in directing proteolytic cleavage of an inventive peptide. It is noted that biosynthetic inventive peptides may degrade under certain storage conditions, resulting in deamidation at one more positions in the inventive peptide. Methionine residues in the inventive peptides may be susceptible to oxidation, primarily to the sulfoxide. As the other derivatives mentioned above, both desamide inventive peptides and/or sulfoxide inventive peptides may be used to exhibit full biological activity.

Other suitable reagents for derivatizing alpha-amino acid-containing residues include imidoesters such as methyl picolinimidate; pyridoxal phosphate; pyridoxal; chloroborohydride; trinitrobenzenesulfonic acid; O-methyliosurea; 2,4-pentanedione; and transaminase-catalyzed reaction with glyoxylate.

The terminal amino acid residues of an inventive peptide with their carboxy (C-terminus) and their amine (N-terminus) groups (as well as carboxy or amide amino acid side chain groups, see above) may be present in their protected (e.g. the C terminus by an amide group) and/or unprotected form, using appropriate amino or carboxyl protecting groups. Also, acid-addition salts of the inventive peptide may be provided. Common acid addition salts are hydrohalic acid salts, i.e., HBr, HI, or more preferably, HCl.

PEGylation of terminal or side chain carboxyl groups or the epsilon-amino group of lysine occurring in the inventive peptide, confers resistance to oxidation and is also within the scope of the present invention

Other modifications resulting in derivatives of inventive peptides are based on carbohydrates and/or lipids which may be covalently coupled to the inventive peptide. It is preferred to couple lipids and/or carbohydrates to serine, threonine, asparagine, glutamine or tyrosine or glutamate or aspartate via their reactive side chain moieties. Alternatively, carbohydrates and/or lipids may also be linked to the terminal moieties of the inventive peptide. Furthermore, an inventive peptide may be coupled to a functionally different peptide or protein moiety, which may also stabilize the inventive peptide and/or may serve to improve the transport properties of an inventive peptide in body fluids, in particular blood. Suitable peptides or proteins may e.g. be selected from Albumin, Transferrin etc., which are directly coupled (as component IV) to the inventive peptide or via a peptide or organic linker sequence. Preferably, these peptides or protein are linked to one of the termini of the inventive peptide.

In order to circumvent the problem of degradation of the inventive peptide another embodiment of the present invention provides a retro-inverso isomer of the inventive peptide composed of D amino acids or at least partially composed of D amino acids. The term "retro-inverso isomer" refers to an isomer of a linear peptide in which the direction of the sequence is reversed and the chirality of each amino acid residue is inverted (see, e.g., Jameson et al., Nature, 368, 744-746 (1994); Brady et al., Nature, 368, 692-693 (1994)). With respect to the parent peptide, the retro-inverso peptide is assembled in reverse order of amino acids, typically with F-moc amino acid derivatives. Typically, the crude peptides may be purified by reversed phase HPLC.

Other modifications, which may be introduced into the inventive peptides relate to modifications of the peptide backbone. Preferably, the modified inventive peptides are scaffold mimetics. Their backbone is different from the natural occurring backbone, while their side-chain structures are identical with the inventive peptides or their fragments, variants, derivatives or analogs. In general, scaffold mimetics exhibit a modification of one or more of the backbone chain members (NH, CH, CO), either as substitution (preferably) or as an insertion. Substituents are e.g. (I) -O-, -S-, or -CH₂- instead of -NH-; (II) -N-, C-Alkyl-, or -BH- instead of -CHR- and (III) -CS-, -CH₂-, -SOₙ-, -P=O(OH)-, or -B(OH)- instead of -CO-. A peptide mimetic of an inventive peptide may be a combination of each of these modifications. In particular, modifications of each the groups I, II and III may be combined. In a peptide mimetic each backbone chain member may be modified or, alternatively, only a certain number of chain members may be exchanged for a non-naturally occurring moiety. Preferably, all backbone chain members of an inventive peptide of either -NH-, -CHR- or CO are exchanged for another non-naturally occurring group. In case the amide bond (-NH-CO-) of the inventive peptide backbone is substituted (in the entire molecule or at least in one single position), preferable substitution moieties are bioisosteric, e.g. retro-inverse amide bonds (-CO-NH-), hydroxyl ethylene (-CH(OH)-CH2-), alkene (CH2=CH-), carba (CH2-CH2-) and/or -P=O(OH)-CH2-). Alternatively, backbone chain elongation by insertions may occur in a scaffold mimetic of the inventive peptide, e.g. by moieties flanking the C-alpha atom. On either side of the C-alpha atom e.g. -O-, -S-, -CH-, -NH- may be inserted.

Particularly preferred are oligocarbamate peptide backbone structure of the inventive peptides. The amide bond is replaced by a carbamate moiety. The monomeric N-protected amino alkyl carbonates are accessible via the corresponding amino acids or amino alcohols. They are converted into active esters, e.g. p-nitro phenyl ester by using the F-moc moiety or a photo sensitive nitroatryloxycarbonyl group by solid phase synthesis.

Inventive peptides are protected against proteolytic cleavage as outlined above. They are in particular protected against dipeptidyl aminopeptidase-4 (DPP-IV). The term "DPP-IV protected" as used herein refers to a peptide according to claim 1. Inventive peptides as well as their derivatives, analogs, fragments and variants render GLP-1(7-35, 36 or 37) as part of component (I) and/or (III) of the inventive peptide resistant to the plasma peptidase (DPP-IV).

Resistance of a peptide to degradation by dipeptidyl aminopeptidase IV is determined e.g. by the following degradation assay: Aliquots of the peptides are incubated at 37 C with an aliquot of purified dipeptidyl aminopeptidase IV for 4-22 hours in an appropriate buffer at pH 7-8 (buffer not being albumin). Enzymatic reactions are terminated by the addition of trifluoroacetic acid, and the peptide degradation products are separated and quantified using HPLC or LC-MS analysis. One method for performing this analysis is: The mixtures are applied onto a Zorbax300SB-C18 (30 nm pores, 5 µm particles) 150 x 2.1 mm column and eluted at a flow rate of 0.5 ml/min with a linear gradient of acetonitrile in 0. 1% trifluoroacetic acid (0%-100% acetonitrile over 30 min). Peptides and their degradation products may be monitored by their absorbance at 214 nm (peptide bonds) or 280 nm (aromatic amino acids), and are quantified by integration of their peak areas. The degradation pattern can be determined by using LC-MS where MS spectra of the separated peak can be determined. Percentage intact/degraded compound at a given time is used for estimation of the peptides DPP-IV stability.

An inventive peptide is defined as DPP-IV stabilised when it is 10 times more stable than the GLP-1 (7-37) based on percentage intact compound at a given time. Thus, a DPP-IV stabilised inventive peptide is preferably at least 10, more preferably at least 20 times more stable than GLP-1 (7-37) as such. Stability may be assessed by any method known to the skilled person, e.g. by adding DPP-IV to a solution of the peptide to be tested and by determining the degradation of the peptide, e.g. over time, by e.g. a spectroscopic method, Western-Blot analysis, antibody screening etc. In parallel, an inventive peptide is defined as a compound, which exerts the effect of GLP-1 (7-37) by e.g. binding to its native receptor (GLP-1 receptor). Preferably, an inventive peptide has a binding affinity to the GLP-1 receptor, which corresponds to at least 10%, preferably at least 50% of the binding affinity of the naturally occurring GLP-1 peptide. The binding affinity may be determined by any suitable method, e.g. surface plasmon resonance etc. Moreover, it is preferred, if the inventive peptide evokes formation of intracellular cAMP by its binding to its extracellular receptor, which transmits the signal into the cell.

The peptides of the invention may be produced synthetically, using solid phase peptide synthesis techniques, similar to the manner of production of GLP-1 (7-36) amide and GLP-1 (7-37) in the art and can be purified afterwards on a laboratory scale e.g. by a single purification step on a reversed-phase HPLC column or suitable chromatography methods.

However, it is preferably formed in engineered cells, either in microbial cells or in animal cell lines to produce the inventive peptide. The inventive peptide may be isolated from the cells from which it is expressed, for instance using conventional separation techniques.

Thus cells may be grown under appropriate conditions, for instance including support and nutrients, in vitro, and secreted protein, i.e. the inventive peptide, is recovered from the extracellular medium. The sequences engineered into cells thus preferably include leader sequences and signal peptide sequences directing secretion of the inventive peptide. The cells preferably express protease capable of cleaving the leader and signal sequences, either endogenously or by engineered gene sequences. In an alternative, the engineered gene sequences encoding an inventive peptide do not include such leader and signal peptide sequences, whereby the intracellularly expressed inventive peptide will not be secreted, and is recovered from cells by processes involving cell lysis. In such methods the coding sequences may include purification tags allowing efficient extraction of the product peptide from the medium, which tags may be cleaved to release isolated inventive peptide.

The invention further provides a nucleic acid, which codes for the inventive peptide, be it a fusion peptide or a fragment, analog or variant thereof. Any nucleic acid coding for an inventive peptides is encompassed by the present invention. Due to degeneracy of the genetic code a plurality of nucleic acid sequences may code for an inventive peptide. A nucleic acid molecule within the scope of the present invention may also contain the nucleic acid coding for the inventive peptide and, additionally, further (functional) nucleotide sequences. In a preferred embodiment of the present invention such a nucleic acid molecule may code (a) for the entire GLP-1 aa sequence (GLP-1(1-37) or the functional GLP-1(7-35, 36 or 37) sequence, (b) a cleavage sequence at the N-terminus of the GLP-1 sequence according to (a) for any protease, upstream from (b) may code for a leader sequence. In another preferred embodiment, upstream from the nucleic acid sequence coding for (b) the nucleic acid molecule may additionally comprise (c) a sequence coding for a signal peptide. Alternatively, the inventive nucleic acid molecule may have sequence (c) fused upstream from (a) without any sequence coding for a leader sequence (b) in between. Preferably, the leader sequence and signal peptide sequence are heterologous to preproglucagon.

The invention further provides a vector comprising an inventive nucleic acid (molecule) and other functional components for expression of the inventive nucleic acid (molecule). Typically, the inventive nucleic acid (molecule) will be fused to a promoter sequence and, eventually combined with other regulator sequences, e.g. an enhancer sequence. For replication, the plasmid may contain an origin of replication. In order to select cells transfected with the inventive vector, one or more antibiotic resistance gene(s) (e.g. kanamycin, ampicillin) may be provided in the vector. The vector may be a plasmid that includes bacterial original promoter, and antibiotic resistance genes and origin promoter, and antibiotic resistance genes for replication and expression in mammalian cells. The invention further provides a host cell comprising exogenously introduced DNA of the invention capable of translating the said precursor protein. The host cell may be either a prokaryotic host cell or an eukaryotic host cell, e.g. a mammalian cell.

According to a further aspect of the invention there is provided a method of treatment of an animal, preferably a human being, by administration of an inventive peptide comprising components (I) and (II) and eventually component (III). It is also provided corresponding use of such inventive peptides in the manufacture of a product for the treatment or prevention of a disease or condition associated with glucose metabolism. Non-limiting examples of glucose disorder include: diabetes mellitus type I or type II (NIDDM), or insulin resistance, weight disorders and diseases or conditions associated thereto, wherein such weight disorders or associated conditions include obesity, overweight-associated conditions, satiety deregulation, reduced plasma insulin levels, increased blood glucose levels, or reduced pancreatic beta cell mass. Preferably, use of inventive peptides for the manufacture of a medicament for the treatment of type 2 diabetes (NIDDM) is disclosed herewith. As a consequence, the present invention relates to a use of the inventive peptide e.g. for lowering weight of a subject, for reducing satiety of a subject, for post-prandially increasing plasma insulin levels in a subject, for reducing fasting blood glucose level in a subject, for increasing pancreatic beta cell mass in a subject or for treating diabetes type I or II in a subject.

Patients with other diseases or disorders may be treated by inventive peptides, i.e. fusion peptides or its analogs, fragments, variants or derivatives, as well. Inventive peptides may be used for the preparation of a medicament for the treatment of neurodegenerative disorders and diseases or conditions associated thereto and for the treatment of disorders and diseases or conditions associated to apoptosis. The use of the inventive peptide for treating these disorders results from the following: GLP-1 receptors, which are coupled to the cyclic AMP second messenger pathway, are expressed throughout the brains of rodents and humans. The chemoarchitecture of receptor distribution in the brain does not only correlate with a central role for GLP-1 in the regulation of food intake and response to aversive stress. It was also shown that GLP-1 binding at its GLP-1 receptor exerts neurotrophic properties, and offer protection against glutamate-induced apoptosis and oxidative injury in cultured neuronal cells. Furthermore, GLP-1 was shown to modify processing of the amyloid β-protein precursor in cell culture and dose-dependently reduces amyloid β-peptide levels in the brain *in vivo.* GLP-1 is therefore also known as regulator of the central nervous system. Inventive peptides mimicking the biological activity of physiologically active GLP-1 have therapeutic relevance to the treatment of e.g. Alzheimer's disease (AD) and other central and peripheral neurodegenerative conditions (e.g. amyotrophic lateral sclerosis (ALS), Alexander disease, Alper's disease, Ataxia telangiectasia, Canavan disease, Cockayne syndrome, Creutzfeldt-Jakob disease, Multiple Sclerosis, Sandhoff disease, Pick's disease, Spinocerebellar Ataxia, Schilder's disease and Parkinson's disease).

Moreover, it was shown that physiologically active GLP-1 exerts anti-apoptotic action on various cells, e.g. GLP-1 is beneficial to the preservation of mass and function of freshly isolated human islets or other cell types. Insofar, the biologically active inventive peptide may be used to treat disorders, which are caused by cell or tissue apoptosis.

The use of an inventive peptide may be for the manufacture of a composition which is administered exogenously, and comprises the isolated inventive peptide. The resulting composition may be used as well for the treatment of the above disorders. The disorders disclosed herein may also be treated by inventive host cells, nucleic acid (molecules) or vectors or, rather, inventive host cells, nucleic acid (molecules) or vectors may be used for the preparation of a medicament for the treatment of these disorders.

Preparation of formulations which contain inventive peptide sequences as active ingredients is generally well understood in the art, as exemplified by US Patents 4,608,251; 4,601,903 ; 4,599,231; 4,599,230; 4,596,792; and 4,578,770, all incorporated herein by reference. Typically, such formulations are prepared as injectables either as liquid solutions or suspensions, preferably containing water (aqueous formulation) or may be emulsified. The term "aqueous formulation" is defined as a formulation comprising at least 50 % w/w water. Likewise, the term "aqueous solution" is defined as a solution comprising at least 50% w/w water, and the term "aqueous suspension" is defined as a suspension comprising at least 50 % w/w water.

For intravenous, cutaneous or subcutaneous injection, or injection at the site of affliction, the active ingredient will be in the form of a parenterally acceptable aqueous solution which is pyrogen-free and has suitable pH, isotonicity and stability. Liquid pharmaceutical compositions generally include a liquid vehicle such as water. Preferably, the liquid vehicle will include a physiological saline solution, dextrose ethanol or other saccharide solution or glycols such as ethylene glycol, propylene glycol or polyethylene glycol or combinations thereof may be included. Further examples are other isotonic vehicles such as Ringer's Injection or Lactated Ringer's Injection.

If the invention relates to a pharmaceutical formulation comprising an aqueous solution of a compound according to the present invention, and a buffer, wherein said compound is present in a concentration from 0.1 mg/ml or above, and wherein said formulation has a pH from about 2.0 to about 10.0, preferably from about 7.0 to about 8. 5. Preferably, the pH of the formulation is at least 1 pH unit from the isoelectric point of the compound according to the present invention, even more preferable the pH of the formulation is at least 2 pH unit from the isoelectric point of the compound according to the present invention.

Solid forms suitable for solution in, or suspension in, liquid prior to injection may also be prepared. The pharmaceutical formulation may be a freeze-dried formulation, whereto the physician or the patient adds solvents and/or diluents prior to use. In other words, the formulation once prepared, is not immediately administered to a subject. Rather, following preparation, it is packaged for storage in a frozen state, or in a dried form for later reconstitution into a liquid form or other form suitable for administration to a subject. In another embodiment the pharmaceutical formulation is a dried formulation (e.g. freeze-dried or spray-dried) ready for use without any prior dissolution. By "dried form" is intended the liquid pharmaceutical composition or formulation is dried either by freeze drying (i.e. lyophilization ; see, for example, Williams and Polli (1984) J. Parenteral Sci. Technol. 38: 48-59), spray drying (see Masters (1991) in Spray-Drying Handbook (5th ed; Longman Scientific and Technical, Essez, U. K.), pp. 491-676; Broadhead et al. (1992) Drug Devel. Ind. Pharm. 18: 1169-1206; and Mumenthaler et al. (1994) Pharm. Res. 11: 12-20), or air drying (Carpenter and Crowe (1988) Cryobiology 25: 459-470; and Roser (1991) Biopharm. 4: 47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

It is possible that other ingredients may be present in the peptide pharmaceutical formulation of the present invention. Such additional ingredients may include wetting agents, emulsifiers, antioxidants, bulking agents, pH buffering agents (e.g. phosphate or citrate or maleate buffers), preservatives, surfactants, stabilizers, tonicity modifiers, cheating agents, metal ions, oleaginous vehicles, proteins (e.g. human serum albumin, gelatin or proteins) and/or a zwitterion (e.g. an amino acid such as betaine, taurine, arginine, glycine, lysine and histidine).

With regard to stabilizers for inventive formulations these may preferably be selected from the group of high molecular weight polymers or low molecular compounds. In a further embodiment of the invention the stabilizer is selected from polyethylene glycol (e.g. PEG 3350), polyvinylalcohol (PVA), polyvinylpyrrolidone, carboxy-hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention. The pharmaceutical compositions may also comprise additional stabilizing agents, which further enhance stability of a therapeutical active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

With regard to surfactants for inventive formulations these may preferably be selected from a detergent, ethoxylated castor oil, polyglycolyzed glycerides, acetylated monoglycerides, sorbitan fatty acid esters, polyoxypropylene-polyoxyethylene block polymers (e.g. poloxamers such as Pluronie F68, poloxamer 188 and 407, TritonX-100), polyoxyethylene sorbitan fatty acid esters, starshaped PEO, polyoxyethylene and polyethylene derivatives such as alkylated and alkoxylated derivatives (tweens, e.g. Tween-20, Tween-40, Tween-80 and Brij-35), polyoxyethylenehydroxystearate, monoglycerides or ethoxylated derivatives thereof, diglycerides or polyoxyethylene derivatives thereof, alcohols, glycerol, lecitins and phospholipids (e.g. phosphatidyl serine, phosphatidyl choline, phosphatidyl ethanolamine, phosphatidyl inositol, diphosphatidyl glycerol and sphingomyelin), derivates of phospholipids (e.g. dipalmitoyl phosphatidic acid) and lysophospholipids (e.g. palmitoyllysophosphatidyl-L-serine and 1-acyl-sn-glycero-3-phosphate esters of ethanolamine, choline, serine or threonine) and alkyl, alkoxyl (alkyl ester), alkoxy (alkyl ether)-derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (e.g. cephalins), glyceroglycolipids (e.g. galactopyransoide), sphingoglycolipids (e.g. ceramides, gangliosides), dodecylphosphocholine, hen egg lysolecithin, fusidic acid derivatives (e.g. sodium tauro-dihydrofusidate etc.), long-chain fatty acids and salts thereof C6-C12 (e.g. oleic acid and caprylic acid), acylcarnitines and derivatives, N'X-acylated derivatives of lysine, arginine or histidine, or side-chain acylated derivatives of lysine or arginine N-acylated derivatives of dipeptides comprising any combination of lysine, arginine or histidine and a neutral or acidic amino acid, N-acylated derivative of a tripeptide comprising any combination of a neutral amino acid and two charged amino acids, DSS (docusate sodium, CAS registry no [577-11- 7]), docusate calcium, CAS registry no [128-49-4]), docusate potassium, CAS registry no [7491-09-0]), SDS (sodium dodecyl sulfate or sodium lauryl sulfate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N, N-dimethyl-3-ammonio-1-propanesulfonate, anionic (alkyl-aryl-sulphonates) monovalent surfactants, zwitterionic surfactants (e.g. N-alkyl-N, N-dimethylammonio-1-propanesulfonates, 3-cholamido-1-propyldimethylammonio-1-propane-sulfonate, cationic surfactants (quaternary ammonium bases) (e.g. cetyl-trimethylammonium bromide, cetylpyridinium chloride), non-ionic surfactants (e.g. Dodecyl-D-glucopyranoside), poloxamines (e.g. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention. The use of a surfactant in pharmaceutical compositions is well-known to the skilled person. For convenience reference is made to Remington: The Science and Practice of Pharmacy, 19th edition, 1995.

With regard to pharmaceutically acceptable preservative these may preferably be selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, ethanol, chlorobutanol, and thiomerosal, bronopol, benzoic acid, imidurea, chlorohexidine, sodium dehydroacetate,chlorocresol, ethyl p-hydroxybenzoate, benzethonium chloride, chlorphenesine (3p-chlorphenoxypropane-1,2-diol) or mixtures thereof.

With regard to isotonic agents these may preferably be selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-solubleglucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galacititol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. With regard to cheating agents these may preferably be selected from salts of ethylenediaminetetraacetic acid (EDTA), citric acid, and aspartic acid, and mixtures thereof.

With regard to buffers these are preferably selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethane, hepes, bicine, tricine, malic acid, succinate, maleic acid, fumaric acid, tartaric acid, aspartic acid or mixtures thereof. Each one of these specific buffers constitutes an alternative embodiment of the invention.

The use of all of the afore-mentioned additives in pharmaceutical compositions containing the inventive therapeutic peptide is well-known to the skilled person, in particular with regard to concentration ranges of the same. For convenience reference is made to Remington: The Science and Practice of Pharmacy, 19th edition, 1995.

The formulations containing the inventive peptide are conventionally administered parenterally, by injection, for example, either subcutaneously, intradermally, subdermally or intramuscularly. A composition for parenteral administration of the inventive peptide may, for example, be prepared as described in WO 03/002136.

Additional formulations which are suitable for other modes of administration include suppositories and, in some cases, oral, buccal, sublinqual, intraperitoneal, intravaginal, anal and intracranial formulations. For suppositories, traditional binders and carriers may include, for example, polyalkalene glycols or triglycerides; such suppositories may be formed from mixtures containing the active ingredient in the range of 0.5% to 10%, preferably 12%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%.

As mentioned above, additional pharmaceutical methods may be employed to control the duration of action. Controlled release preparations may be achieved by the use of polymers to complex or absorb a peptide of the present invention. The controlled delivery of the active ingredient (peptide) may be exercised by selecting appropriate macromolecules (for example, polyesters, polyamino acids, polyvinylpyrrolidone, ethylene vinylacetate copolymers, methylcellulose, carboxymethylcellulose, and protamine sulfate), the concentration of the macromolecules as well as the methods of incorporation. Such teachings are disclosed in Remington's Pharmaceutical Sciences (see above). Another possible method to control the duration of action by controlled release preparations, is to incorporate a peptide of the present invention into particles of a polymeric material such as polyesters, polyamino acids, hydrogels, poly(lactic acid) or ethylene vinylacetate copolymers.

The inventive peptides may be formulated as neutral or salt forms. A peptide of the present invention may be sufficiently acidic or sufficiently basic to react with any of a number of organic and inorganic bases, and organic and inorganic acids, to form an (addition) salt, e.g. formed with the free amino groups of the peptide. Acids commonly employed to form acid addition salts are inorganic acids such as hydrochloric acid, hydrobromic acid, hydroiodic acid, sulfuric acid, phosphoric acid, and the like, and organic acids such tartaric, asp-toluenesulfonic acid, methanesulfonic acid, oxalic acid, mandelic, p-bromophenyl-sulfonic acid, carbonic acid, succinic acid, citric acid, benzoic acid. Examples of such salts include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleat, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolat, tartrate, methanesulfonate, propanesulfonate, Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like. Acid addition salts, carboxylate salts, lower alkyl esters, and amides of the inventive peptides may be formulated according to WO 91/11457 (1991); EP 0 733 644 (1996); and U.S. 5,512,549 (1996).

Formulations containing the inventive peptide sequences are administered in a manner compatible with the dosage formulation, and in such amount as will be therapeutically effective. The quantity to be administered depends on the subject to be treated, including, e.g., the severity of the patient's disease. Suitable dosage ranges are e.g. of the order of several hundred micrograms active ingredient per therapeutic dose with a preferred range from about 0.1 µg to 2000 µg (even though higher amounts in the 1-10 mg range are contemplated), such as in the range from about 0.5 µg to 1000 µg, preferably in the range from 1 µg to 500 µg and especially in the range from about 10 µg to 100 µg.

Formulations containing the inventive peptides plus e.g. additional excipients, e.g., glycine and mannitol or other additives, may be marketed in a lyophilized form as vials. A companion diluent vial is provided, allowing the patient to reconstitute the product to the desired concentration prior to administration of the dose. Inventive formulations can also be marketed in other well known manners, such as prefilled syringes, etc.

The invention is illustrated further in the accompanying examples.

### Examples

### Example 1

### Creation of genetic constructs

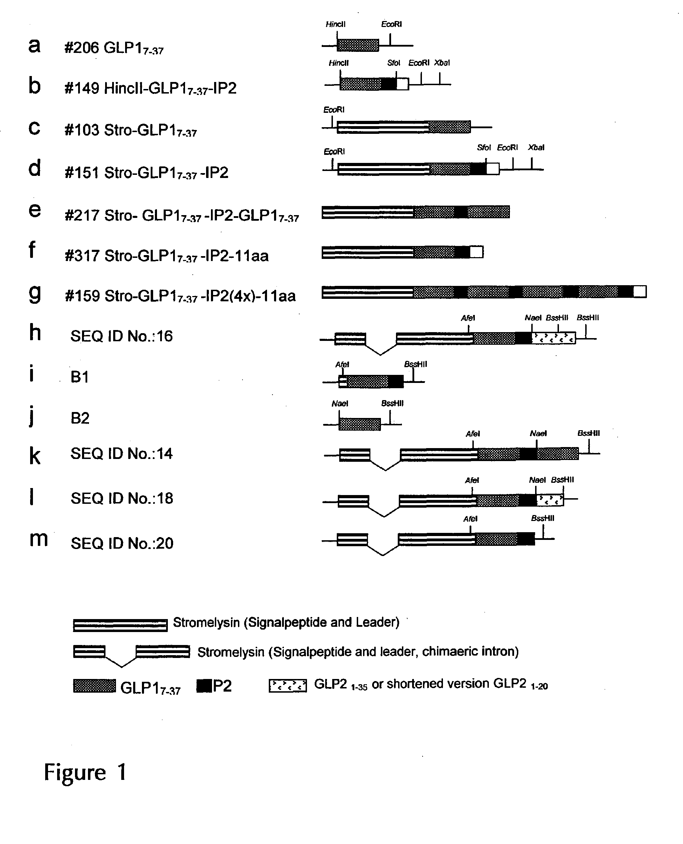

The coding sequence for GLP-1 (7-37) cDNA was synthesised synthetically, in a sequence including HincII and EcoRI sites as indicated in Fig. 1a. Separately the cDNA illustrated in Fig. 1b was synthesised, including the coding sequences for GLP-1(7-37), IP2 and restriction sites for Sfol, EcoRI and Xbal, as illustrated in Fig. 1b. To direct GLP-1 to the secretory pathway, the heterologous signal sequence of stromelysin 3 (Acc.No. NM_005940) was used. Therefore the cDNA, encoding stromelysin signal and leader sequence was reverse transcriptase PCR amplified from human RNA, and used with the construct of Fig. 1a or Fig. 1b to form the construct shown in Fig. 1c and Fig. 1d, respectively.

The Hincll/EcoRl fragment of the Fig. 1a construct is cloned into the Sfol site of the sequence of Fig. 1d to form the construct Fig. 1e. Similarly, the EcoRI fragment of Fig. 1d is cloned into the EcoRI site of an eukaryotic expression plasmid, to produce the construct shown in Fig. 1f. To form the construct shown in Fig. 1g, the Hincll/Xbal fragment of the construct shown in Fig. 1b is repetitively cloned into the Sfol/Xbal site of the construct shown in Fig. 1d. Figure 1h shows a synthesized, codon optimized sequence encoding the stromelysin leader and signal sequences interrupted by a shortened endogenous intron sequence, fused to sequences encoding human GLP-1(7-37), IP2 and GLP-2(1-35). The DNA sequence of the construct Fig. 1h is SEQ ID No.:16, while SEQ ID No.:15 also shows the sequence of the translated peptide.

Also synthesised are the sequences in Figs 1i and 1j. These are then used to form the construct in Fig. 1k, by cloning the Nael/BssHll fragment of Fig. 1j into the Nael/BssHll linearised sequence of Fig. 1h. The DNA sequence of the construct Fig. 1k is SEQ ID No.: 14, while SEQ ID No.:13 also shows the sequence of the translated peptide. The construct of Fig. 1l is formed by BssHll digest and religation of the sequence of Fig. 1h. The DNA sequence of the construct Fig. 1l is SEQ ID No.: 18, while SEQ ID No.:17 also shows the sequence of the translated peptide. The construct of Fig. 1m is formed by cloning the Afel/BssHll fragment of the sequence of Fig. 1i into the Afel/BssHll linearised sequence of Fig. 1h. The DNA sequence of the construct Fig. 1m is SEQ ID No.: 20, while SEQ ID No.:19 also shows the sequence of the translated peptide.

The above constructs may be made by a person skilled in the art using routine techniques. Inventive alternative fusion peptides having an IP2 homologue (as component (II)) as definded by general formula (I) may be constructed analogously. Accordingly, inventive fusion peptides were constructed having the sequence (RR)DFLEEVAIAEELG, (RR)DFIEEVAIAEELG, (RR)DFPEEVAIAEEIG, (RR)DFLEEVAIAEEIG as component (II).

### Example 2

### Western Blot Analysis of GLP-1 peptides

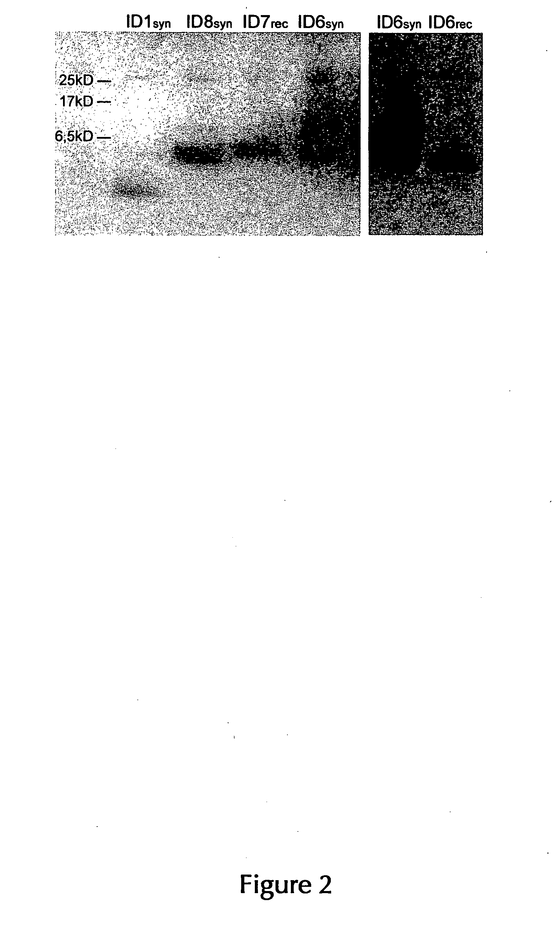

Various GLP-1 peptides were produced synthetically by solid phase (*syn*) or recombinant using E.coli (rec). GLP-1 peptides (31 ng SEQ ID No:1 and 10 ng of each SEQ ID No:6, SEQ ID No:7, SEQ ID No:8) were separated in a 10%-20% gradient SDS PAGE (120V, 90minutes) and transferred to a PVDF membrane (Immobilon-P Membran 0,45 µm Millipore IPVH 00010) by semi-dry blotting (2.0 mA/cm², 60 minutes). After methanol fixation and blocking (3% (w:v) BSA, 0.1 % (v:v) Tween-20 in TBS) the membrane was immunoblotted with 1 µg/ml anti-GLP-1 antibody (HYB 147-12, Antibodyshop) at 4°C o/n. After washing and incubation with 0.02 µg/ml detection antibody (Anti Mouse IgG, HRP conjugated, Perkin Elmer PC 2855-1197) at RT for 4 hours, chemiluminescence detection reveals the location of the protein. Fig. 2 shows a Western Blot for the peptides indicated. The following values can be given: SEQ ID No.: 1 (ID1syn) corresponds to GLP-1(7-37), 31 aa, 3,3 kD; SEQ ID No.:8 (ID8 syn, CM3) corresponds to GLP-1(7-37)-IP2, 46 aa, 5,1 kD; SEQ ID No.: 7 (ID7rec, CM2) corresponds to GLP-1(7-37)-IP2-RR-GLP2, 83 aa, 9,4 kD; SEQ ID No.: 6 (ID6syn, CM1) corresponds to GLP-1(7-37)-IP2-RR-GLP1(7-37), 79 aa, 8,7 kD.

### Example 3

### In vitro human plasma stability of GLP-1^{CM} peptides

Synthetic GLP-1 peptides (SEQ ID No:1_{syn,} SEQ ID No:6_{syn,} SEQ ID No:7_{rec,} SEQ ID No:8_{syn}) were incubated at concentrations of 20 ng/ml with human plasma at 37°C and 5% CO₂ for 3 hours. Dipeptidylpeptidase activity of the plasma was inhibited by an DPP-IV inhibitor (#DPP4, Biotrend). Active GLP was measured using the GLP-1 (Active) ELISA (#EGLP-35K, Biotrend).

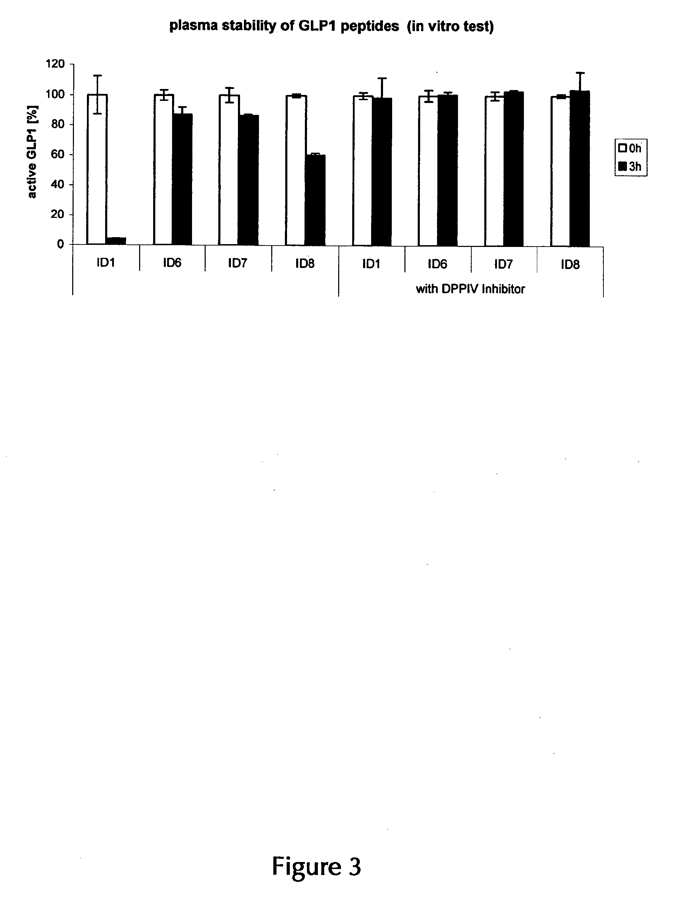

In contrast to the native GLP-1₍₇₋₃₇₎ (SEQ ID No:1) the inventive C-terminal elongated GLP-1 peptides SEQ ID No:6, SEQ ID No:7, and SEQ ID No:8 are significantly stabilized in human plasma in vitro (Fig. 3). As control (on the right hand side) the results obtained for experiments with addition of DPP-IV are shown. GLP-1 activity is completely maintained in these control experiments.

### Example 4

### Bioassay in vitro

### Cyclic AMP Production

RIN-5F cells (rat islet cell tumor; ECACC No. 95090402) were grown in 24-well plates for 4 days reaching 70% confluence. Cells were washed twice with DMEM (E15-009, PAA) before addition of 0.5 ml DMEM (E15-009, PAA) supplemented with 1% HSA (Aventis), 0.2 mM IBMX (858455, Sigma) and the test peptides. After a 20 minute incubation at 25°C, cells were washed twice with ice cold PBS. Cellular cAMP was extracted by addition of 0.1 N HCI containing 0.5% Triton X-100. Cyclic AMP was quantified using the cAMP (low pH) EIA (Cat. DE0355, R&D). For stimulation 3*10⁻⁸ M SEQ ID No:1, SEQ ID No:6_{syn}, SEQ ID No:6_{rec}, SEQ ID No:7_{rec}, SEQ ID No:8_{syn} have been used.

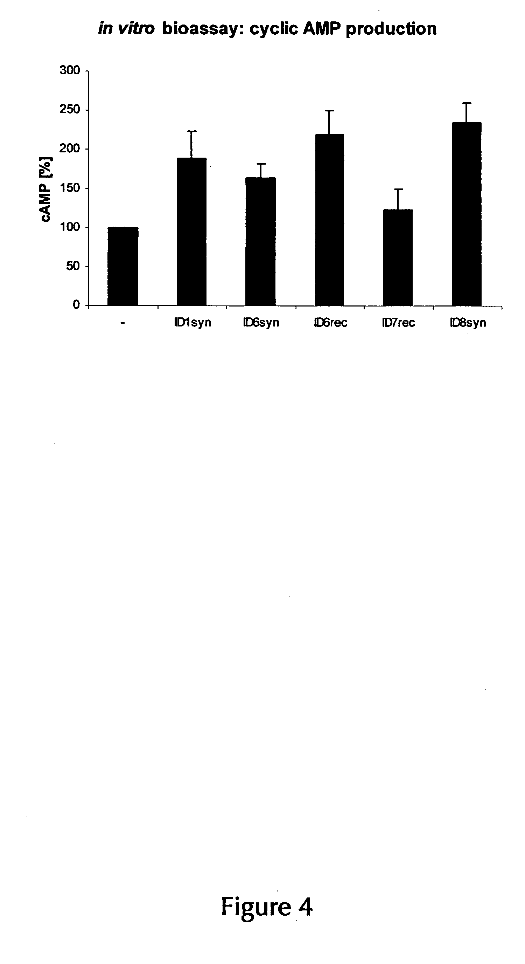

Results are shown in Fig. 4. 100% cAMP production corresponds to the basal production in the absence of GLP-1. GLP-1 binds to G protein-coupled receptors and stimulates cAMP production. All molecules tested increase the cellular cAMP production.

### Example 5

### in vivo bioactivity

11-week-old type II diabetic mice (C57BL/Ks-Lepr^{db/db}, Harlan) were treated with 5 µg peptide by subcutaneous injection twice a day at 9 a.m. and 5 p.m. (n=5 per group). Blood glucose was measured before (day 0) and after treatment with GLP^{CM} peptides (Day 2, 4, 7, 10) at 10 a.m. after an overnight fastening period. Data were presented in relation to blood glucose levels measured at day 0.

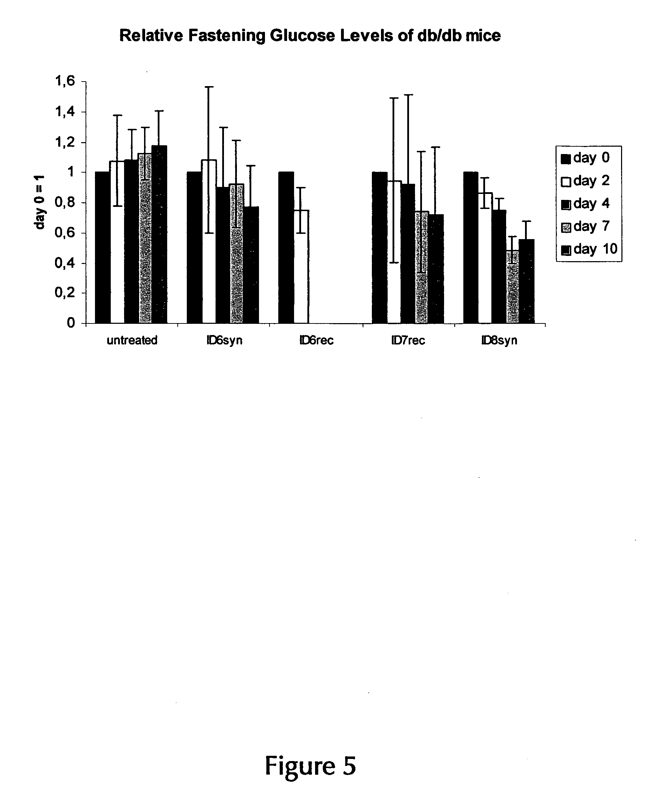

All inventive peptides tested (SEQ ID No.:6 (synthetic or recombinant) and SEQ ID No.:7 (synthetic or recombinant)) have an anti-hyperglycemia effect. Best results were obtained with recombinant SEQ ID No.:6 (CM1) and synthetic SEQ ID No.:8 (CM3). In Fig. 5 (y-axis) the relative effect of the treatment is shown. Blood glucose at day = 0 was set to 1. Untreated animals undergo continuous increase in blood glucose level over time, whereas animals treated with inventive peptides display *grosso modo* a continuous decrease of the blood glucose level over time.

### Example 6

### Measurement of in vitro plasma stability of GLP1^{CM} peptides (kinetic test method)

Aliquots of 1 µM peptide in incubation buffer (50mM Triethanolaminl-HCl (pH 7,8), 0.2% HSA) from CM3, Alanin substituted CM3 analogs (CM3-ANA01, CM3-ANA02, CM3-ANA03), C-terminally shortened CM3 analogs (CM3-ANA06, CM3-ANA07) or C-terminally elongated CM3 analogs (CM3-ANA09) were incubated with 10% human plasma at 37°C and 5% CO₂ for 0, 3, 6 and 9 hours. Dipeptidylpeptidase activity was stopped by addition of DPPIV inhibitor (#DPP, Biotrend) and active GLP-1 levels determined using the GLP-1 (active) ELISA (#EGLP-35K, Linco). Results are from triplicates in at least two independent experiments.

Inventive peptides having at least nine amino acids added to the C-terminus of GLP-1 are CM3 (murine, GLP-1(7-37)-IP2) and derivatives thereof with modified sequences in component (II) (IP2), i.e. CM3-ANA01, CM3-ANA01, CM3-ANA02, CM3-ANA03, CM3-ANA05, CM3-ANA07, CM3-ANA07. Peptides GLP-1 and CM3-ANA06 reflect control or reference substances.

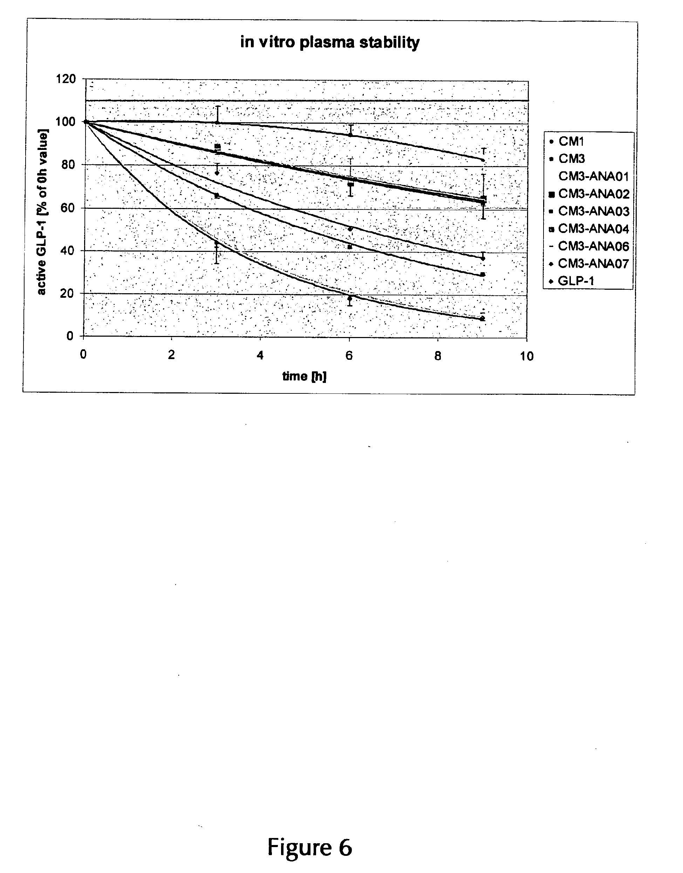

Fig. 6 shows the active portion of active GLP-1 as a % of the 0h value (0h = 100%). It is clearly seen that GLP-1 has the worst plasma stability with only 9% left after 9h plasma exposure. CM3-ANA01 shows the best stability values with 84% material left after 9h. Inventive peptides have a remainder of at least 30% after 9h, whereas peptides with shorter extensions do not reach these values. From the kinetics, a time can be determined, which is needed to degrade active GLP-1 to 80% of the 0h value (DT₈₀: 80% degradation time) for the substances tested.

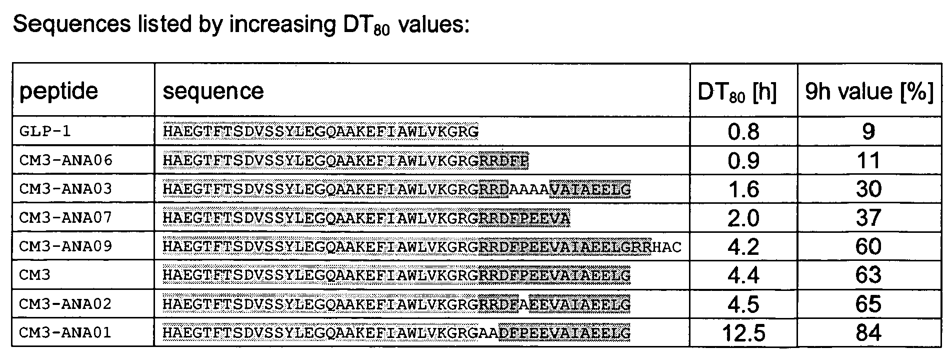

The inventive substances show a considerably longer in vitro plasma stability as compared to the reference substances. Without being bound to any theory, the C-terminal elongation of GLP-1 introduces a steric hindrance, preventing these analogs to enter the active site of the DPPIV, and thus escape degradation, whereas shorter reference substances do not show this protecting effect for GLP-1 stability. This hypothesis is supported by the continuously increasing stability of cumulative C-terminal elongations of the peptides CM3-ANA06 (36aa), CM3-ANA07 (40aa) and CM3 (46aa). Nevertheless this phenomenon is not only dependent on the pure number of amino acids, which has been shown by Alanin substitutions in the IP2 region of CM3. The CM3-ANA03 peptide, which has the same number of amino acids like CM3, has a strongly reduced stability compared to CM3. On the other hand CM3-ANA01, which also consists of 46 amino acids, has a higher plasma stability than CM3. This may indicate that beside the number of amino acids, additional steric effects may influence the stability. Particularly preferred are peptides having an elongation comprising IP2 at the C-terminus of GLP-1 or an elongation having a certain degree of homology with IP2, which may result from a specific conformation, which hinders the N-terminal region from entering the active site of DPPIV.

### Example 7

### In vivo study - Immunogenicity

To elucidate potential immunogenicity of test substance CM1 a mouse study was performed at Parabioscience (Groningen, Germany). The trial was done in BALB/c mice receiving 5 repeated injections (70µg peptide / dose, i.v.) over 22d (n=5).

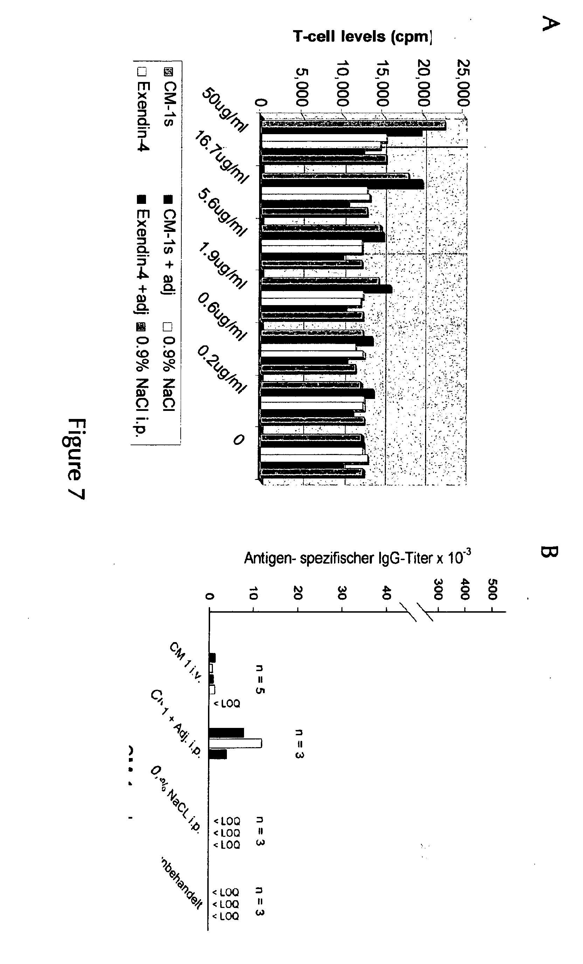

CM1_{syn} induced no toxicity and only a minimal T-cell antibody response and antibody titer as shown in the following figure. An immunologic effect of by-products (purity of the peptide only 95%) cannot be excluded. Fig. 7 shows studies for evaluation of potential immunogenic effects of CM1 syn. On the left hand side (Fig. 7A), results of a T-cell recall response assay are shown. Spleens of the treated and control mice were explanted, T-cells isolated and stimulated with increasing amounts of antigene. Proliferative recall response is measured. On the right hand side (Fig. 7B), the antigen specific IgG antibody titer in the immune sera of the animals was determined.

### Example 8

### Dose-efficacy study in diabetic mice

After a two-hour fastening period diabetic C57BL/KsJ@Rj-db (db/db) mice were treated with five different concentrations of GLP-1, CM1, CM3, CM3-ANA01 and exendin-4. A sixth group was treated with saline only. 7 animals were treated per group. Blood glucose was determined from tail bleeds directly before, 1 hour and 4 hours after injection.

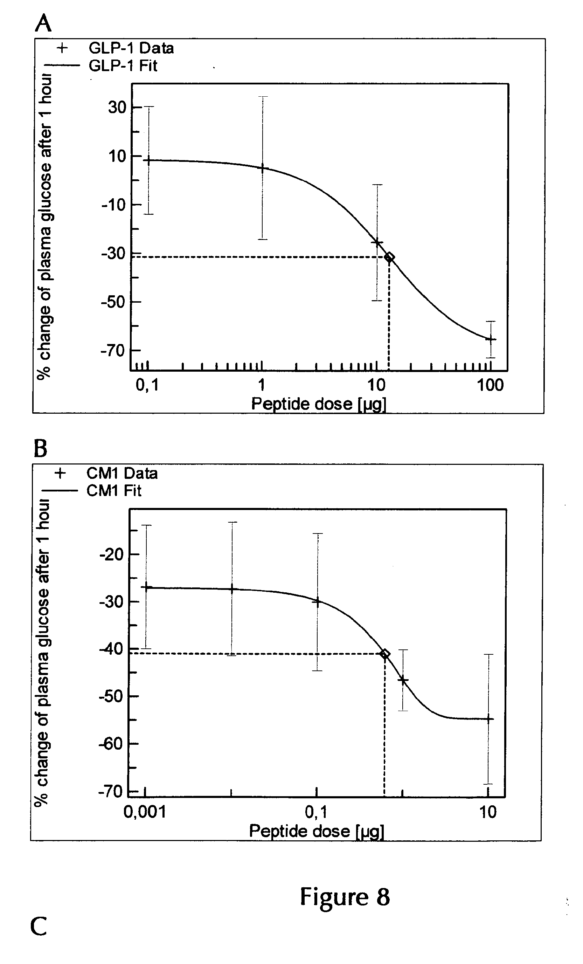

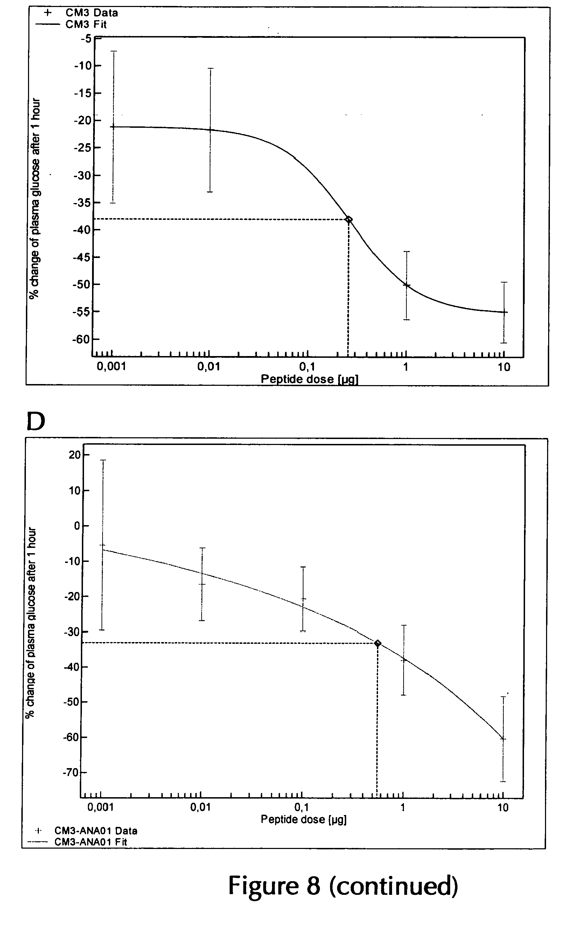

The results are shown in dose-response curves according to Fig. 8 (Fig. 8A (GLP-1), Fig. 8B (CM1), Fig. 8C (CM3) and Fig. 8D (CM3-ANA01)). For every test substance a graph was prepared with the percentage difference of the blood glucose in relation to the start value for the different concentrations. The ED₅₀ value for the different test substances was determined by preparing a dose response curve with the values of the 1 h measurements as a percentage reduction in comparison to the basal value. To evaluate the ED₅₀ value for GLP-1, CM3-ANA01, CM3_{rec} and Exendin-4 the Morgan-Mercer-Flodin (MMF) model was used, for CM1 the Richards model was used. Both models are sigmoidal fit models suitable for dose response evaluation. For every peptide the plasma glucose ED₅₀ values (single s.c. dose in µg / mouse) have been determined from the percentage decrease of blood glucose. They are summarized in table 1.

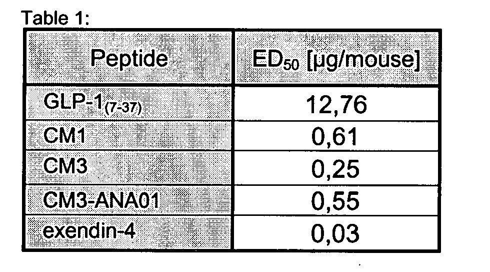

Deduced from the ED₅₀ values, the GLP-1 analogs CM1, CM3 and CM3-ANA01 reveal a more than 20fold better *in vivo* bioactivity, which is most likely a result of the enhanced plasma stability.

All peptides tested lead to a significant decrease of blood glucose levels in hyperglycaemic db/db mice at least for the highest concentrations. The fall in plasma glucose after a single s.c. injection of 1.1 - 3.0 nmol peptide (versus control) is summarized in table 2. Table 2 exhibits the glucose lowering effects of the test substances (versus control). The fall in plasma glucose and the corresponding significance levels are given for the time period 1 hour (@1h) and 4 hours (@4h) after peptide injection. Differences have been observed in the long-term efficacy of the peptides. Only exendin-4 and CM3 revealed a significant decrease of the blood glucose levels after 4 hours.

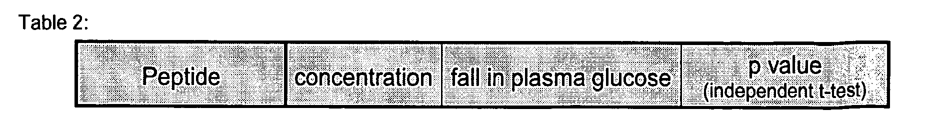

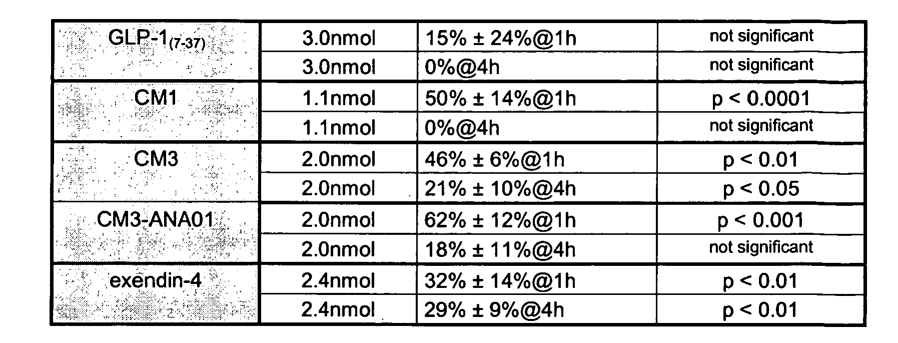

### Example 9

### Long-term treatment of db/db mice

Four groups with n=12 C57BL/KsJ@Rj-db (db/db) mice have been investigated:
- Group A: treatment with vehicle (0.9% saline) once daily
- Group B: treatment with 24nmol/kg test substance 1: CM1 rec once daily
- Group C: treatment with 24nmol/kg test substance 2: CM3-ANA01 once daily
- Group D: treatment with 24nmol/kg reference substance exendin-4 (known in the art and approved (however, exendin-4 is not an GLP-1 analog)) once daily

Peptides or vehicle will be given once daily (group A-D) between 2°° - 3°°p.m. subcutaneously in the skin fold of the back. The regimen was continued for 18 weeks. From week 12 to 18 treatment was done twice a day in 6 of 12 animals per group.

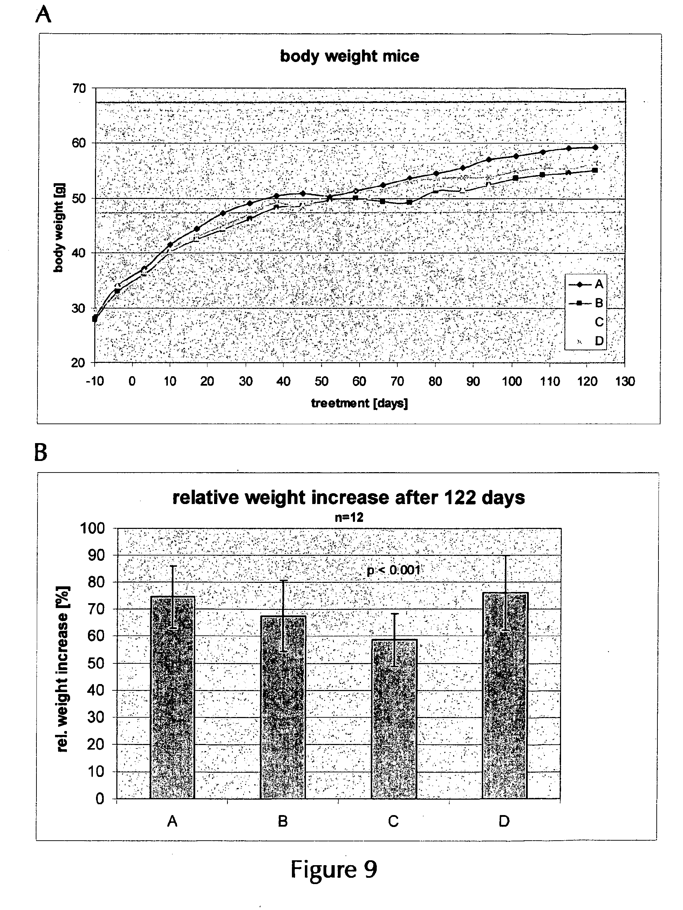

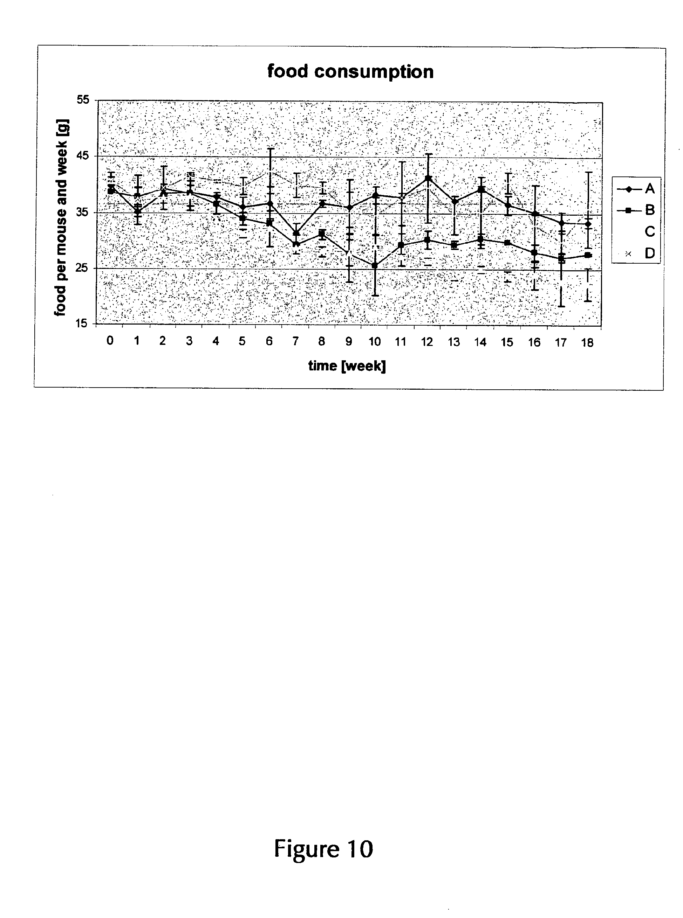

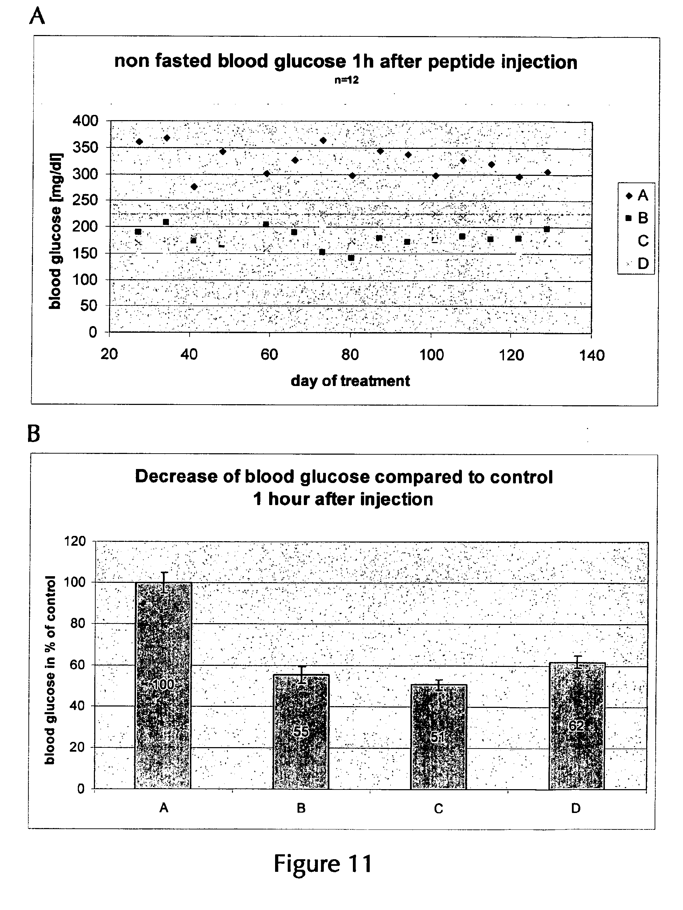

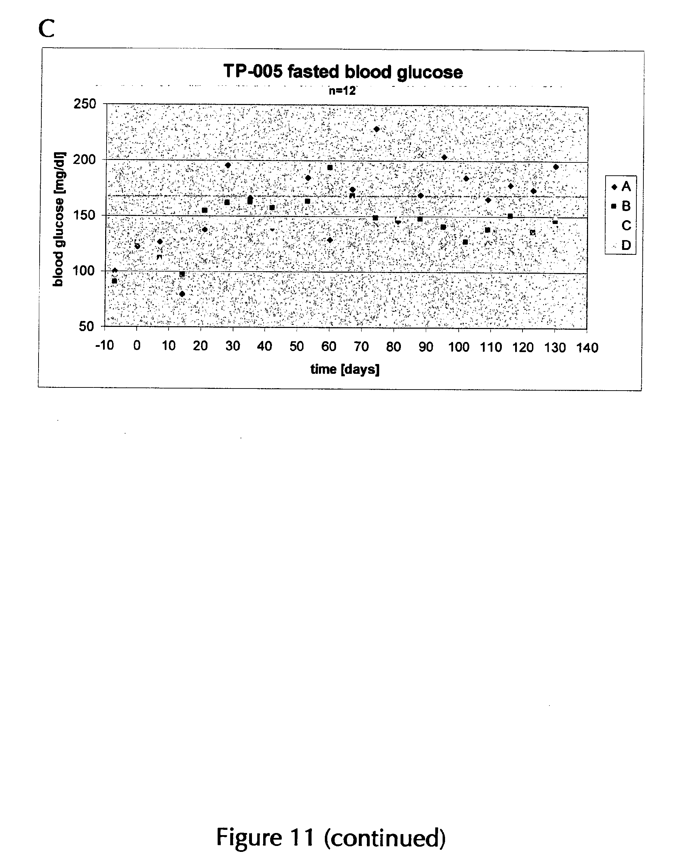

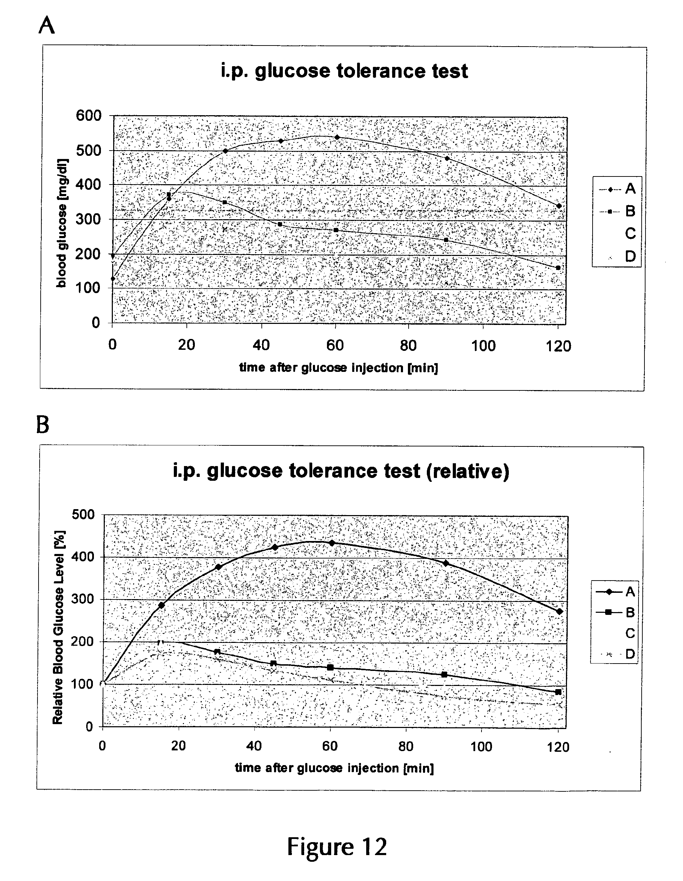

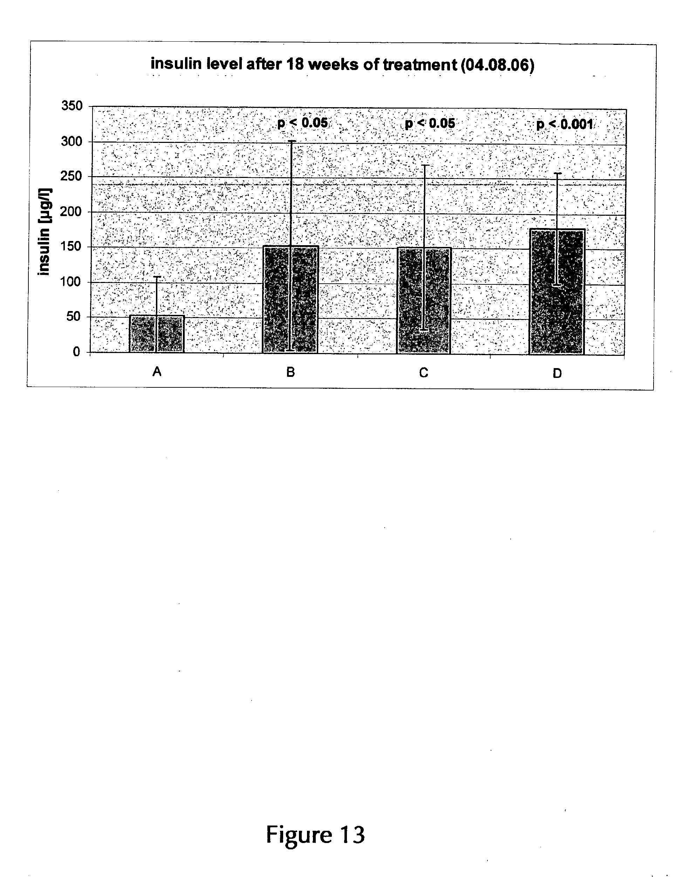

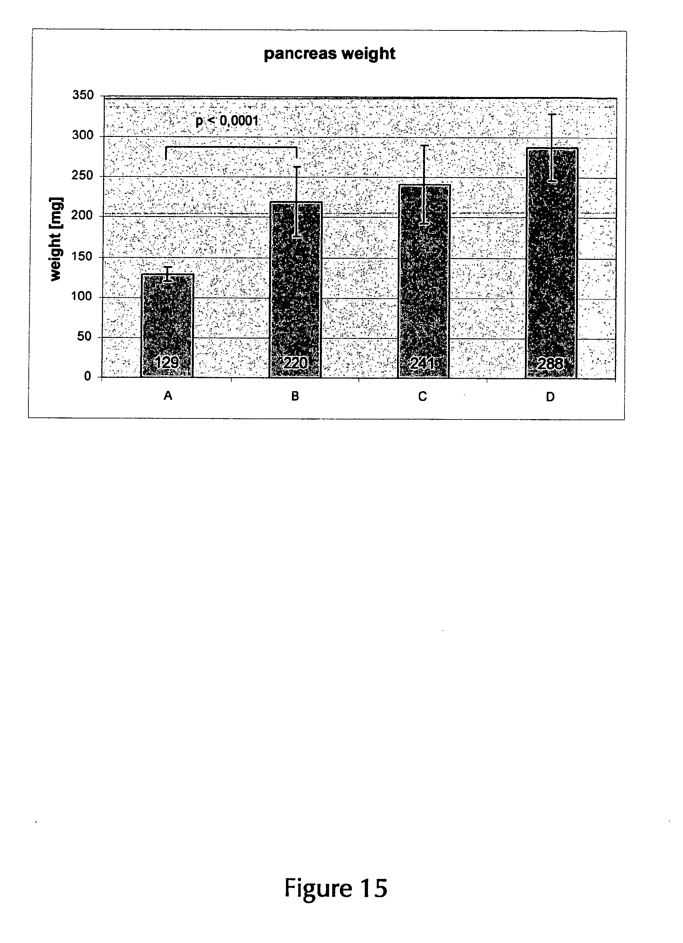

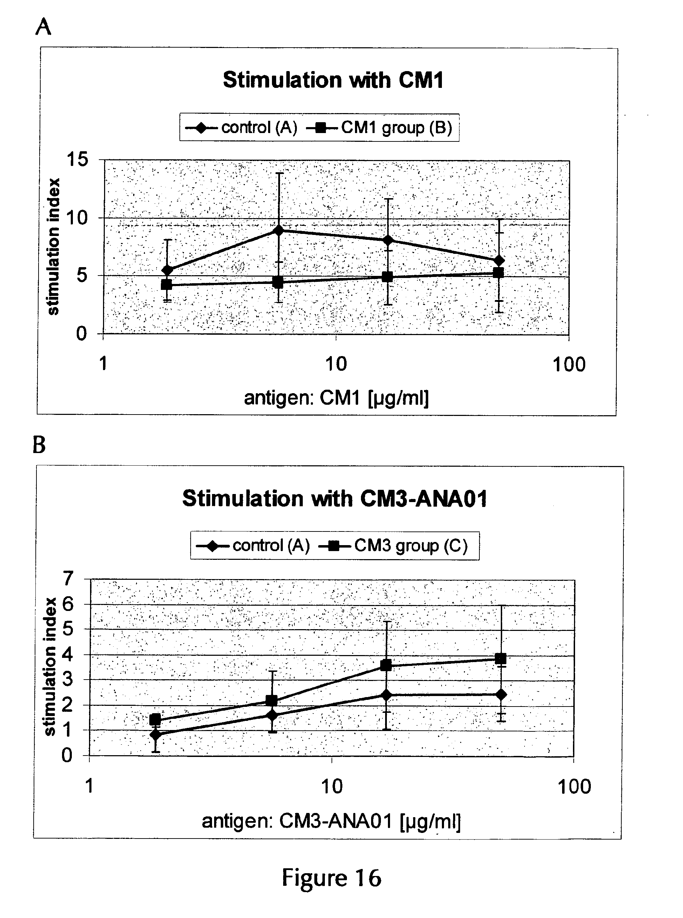

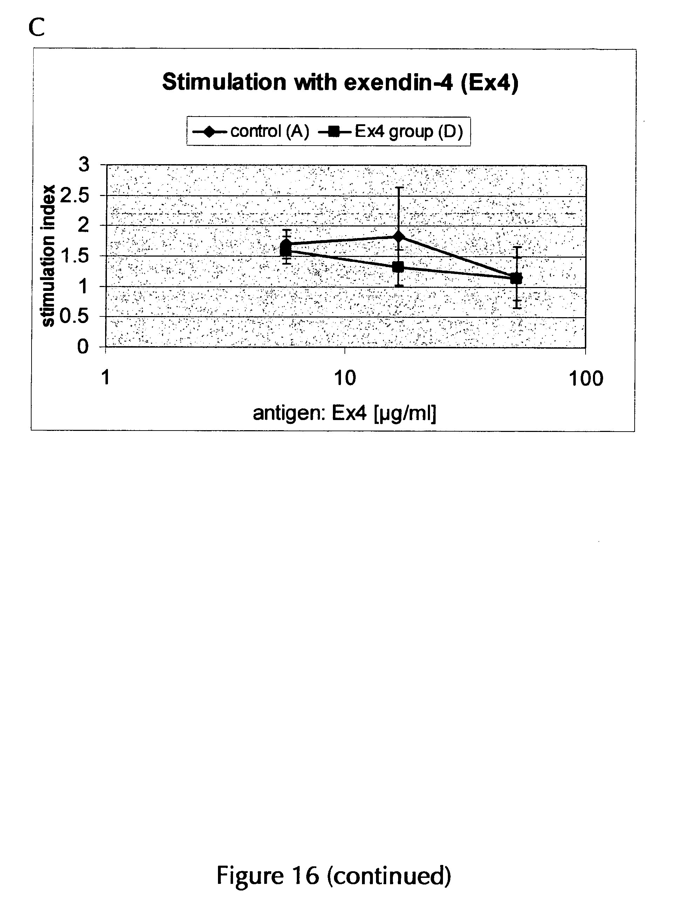

Various parameters of the mice were investigated, i.e. health status (1), body weight (2), food consumption (3), blood glucose (4), glucose tolerance test (5), insulin data (6), glycosylated hemoglobin (7), pathology (8), and restimulation of T cells (9).
Health status (1) was good in all groups, no side effects of the treatment have been seen.
Body weight (2) was lower in all treated groups after 18 weeks of treatment (Fig.9). Relative body weight increase is significantly lower in the CM3-ANA01 group. Fig. 9A shows the effect of a treatment with saline (A), CM1 (B), CM3-ANA01 (C) or exendin (D) on the body weight of db/db mice. The mean values of 12 animals per group are plotted. Fig. 9B shows the effect of a treatment with saline (A), CM1 (B), CM3-ANA01 (C) or exendin (D) on the body weight of db/db mice. The relative body weight increase of 12 animals per group after a treatment of 122 days is plotted. Only the treatment with test substance CM3-ANA01 (group B) revealed a significantly lower weight increase (p<0.001).
Food consumption (3) was significantly lower in the CM1 and CM3-ANA01 group compared to the control. Fig. 10 shows the effect of a treatment with saline (A), CM1 (B), CM3-ANA01 (C) or exendin (D) on the weekly food consumption of db/db mice during a 122 day treatment period. The relative food consumption per mouse is significantly decreased in group B and C (p<0.001).
Blood glucose (4) levels were determined in a variety of situations. *Non-fasted blood glucose level* (1 hour after peptide injection) is shown in Fig. 11A, i.e. the effect of a treatment with saline (A), CM1 (B), CM3-ANA01 (C) or exendin (D) on the non-fasted blood glucose levels. Blood glucose was measured from a tail bleed 1 hour after s.c. injection of saline (A) or the peptides CM1 (B), CM3-ANA01 (C) or exendin (D) in a concentration of 24nmol/kg. Compared to control non fasted blood glucose level 1 h after s.c. peptide injection is reduced to 55% in group B, 51 % in group C and 60% in group D (Fig. 11B). The blood glucose drecrease in the treated groups treated groups is highly significant (p < 0.0001).
   Fasted blood glucose level (18 hours after peptide injection) are shown in Fig. 11C, i.e. the effect of a treatment with saline (A), CM1 (B), CM3-ANA01 (C) or exendin (D) on the fasted blood glucose levels. Blood glucose was measured from a tail bleed after a 12-hour overnight fast 18 hours after s.c. injection of the test substances. Fasted blood glucose level 18h after peptide injection is reduced in the CM1 and CM3-ANA01 group.
An i.p. glucose tolerance test (5) (IPGTT) was conducted after 8 weeks of treatment on mice of the groups A-D. The basal blood glucose value (0 min) of 12 hour fasted animals has been determined by tail bleed followed by an s.c. injection of the test substance and an i.p. injection of 20% glucose solution (1g glucose per kg). Blood glucose has been further on determined 15, 30, 45, 60, 90 and 120 minutes after glucose injection. A significant normalization of glucose tolerance was shown in all treated groups (Fig. 12A). In Fig. 12A absolute blood glucose levels during an i.p. glucose tolerance test (IPGTT) after 8 weeks of treatment with saline (A), CM1 (B), CM3-ANA01 (C) or exendin (D) is shown (mean values of each group (n=12)).
   Another presentation of the data presented by Fig. 12A of the blood glucose tolerance test is given in Fig. 12B. *Relative* blood glucose levels during an i.p. glucose tolerance test (IPGTT) after 8 weeks of treatment with saline (A), CM1 (B), CM3-ANA01 (C) or exendin (D) normalized on the starting blood glucose level (100%).
Insulin levels (6) were determined from mice after a 12 hour overnight fastening period. After 18 weeks of treatment, all mice of the treated groups produce significantly more insulin than the non-treated control. Fig. 13 presents the data for serum insulin levels in mice after 18 week treatment with 0.9% saline (Group A), CM1 peptide (group B), CM3-ANA01 peptide (group C) or exendin-4 (group D). Directly after sampling blood samples have been treated with a protease inhibitor cocktail to avoid insulin degradation. The serum was analysed for insulin with the *Insulin Mouse Ultrasensitive ELISA* (Cat.# EIA-3440, DRG). Significance has been determine with Student's t-test.
Glycosylated hemoglobin (7) is formed by excess plasma glucose binding to hemoglobin in red blood cells (RBC). Since RBCs have a 120-day lifespan, measurements of the glycolylated hemoglobin gives a longer-term indication of glucose control and is seen as a better indicator than plasma glucose levels. Whole blood was collected from the retro-orbital sinus and analyzed with the *Enzymatic HbA1c Test Kit* (#DZ121A, Diazyme) to determine the glycosylated hemoglobin levels. Using glycosylated hemoglobin as a parameter, a significant reduction in the increase was seen in all treated groups.

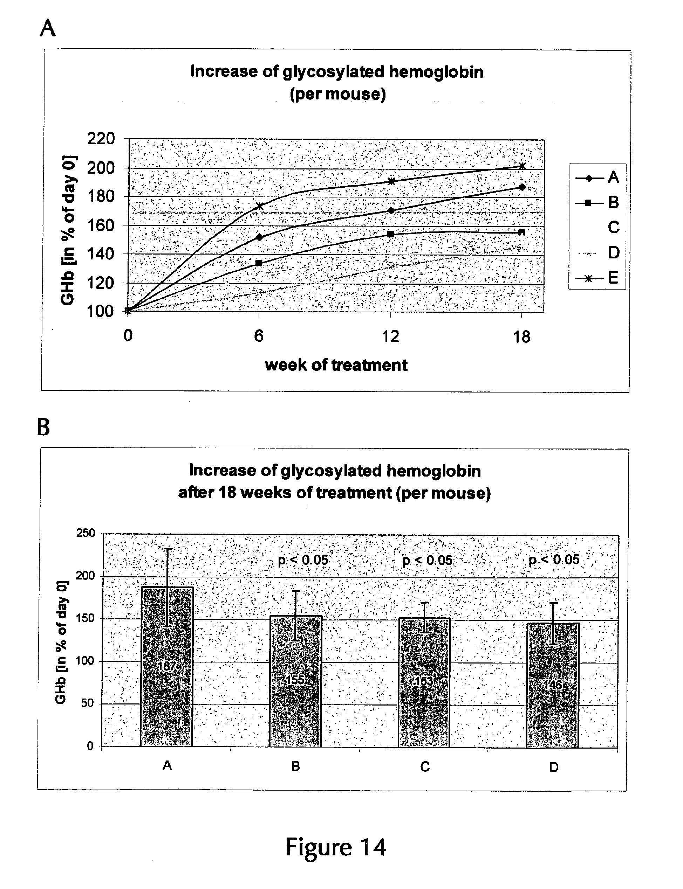

   Fig. 14A shows the relative increase of glycosylated hemoglobin (GHb) in whole blood samples after 6, 12 and 18 weeks of treatment with saline (A), CM1 (B), CM3-ANA01 (C) or exendin (D). Fig. 14B shows the relative increase of glycosylated hemoglobin (GHb) in whole blood samples after 18 weeks of treatment with saline (A), CM1 (B), CM3-ANA01 (C) or exendin (D). The mean value of all animals per group (n=12) is given. The increase of the glycosylated hemoglobin levels of all treated groups are significantly lower than the control.
Pathology (8) of the mice treated was conducted. Necropsy revealed no differences in the organs of the treated groups compared to the non-treated group A, except the pancreas volume. Pancreas weight was determined and revealed to be highly significant higher in all treated groups. Figure 15 presents the pancreas weight determined at day of scarification of the animals after 18 weeks of treatment with 0.9% saline (Group A, n=11), CM1 peptide (group B, n=12), CM3-ANA01 peptide (group C, n=12) or exendin-4 (group D, n=12).
   To evaluate potential immunological effects of the tested substances, type IV immunogenicity (9) was examined by T cell restimulation. Therefore, the spleen of 8 animals per group was explanted, T-cells isolated and restimulated with different concentrations of the corresponding test substance. Compared to non-treated controls no significant increase in peptide restimulated T-cell proliferation was found. Figure 16 shows the *in vitro* recall response of spleen cells to different concentrations of the test substances CM1 (Fig. 16A) and CM3-ANA01 (Fig. 16B) and the reference substance exendin-4 (Fig. 16C). Using a non-radioactive cell proliferation assay (Cell Proliferation ELISA, BrdU, chemiluminescent) the *in vitro* recall response of spleen cells of mice of group A to D to 3-fold dilutions of the test substances CM1 and CM3-ANA01 and the reference substance exendin-4, starting at 50 µg/ml, were measured in triplicates of individual mice. The stimulation index (SI) was calculated as the quotient of the response in the presence of the respective peptide and the response in the absence of peptides. Mean values ± standard deviations of the respective treated group and control group are shown (group A: n=3, group B: n=6, group C: n=7, group D: n=6). For the calculation of the mean values only mice, with a SI of more than 6.5 in the positive control cultures with 5 µg/ml Con A were analysed.

The long-term study in diabetic mice further supports the efficacy of the inventive C-terminally elongated peptides. In all parameters tested (body weight, food consumption, blood glucose levels, glycosylated hemoglobin, glucose tolerance, insulin secretion, pancreas weight) the C-terminally elongated peptides revealed a significant therapeutic effect. Taking in account the homology of the C-terminally elongated CellMed peptides with the endogenously occurring sequence it was shown that these inventive peptides are advantageous in terms of immunogenicity compared to non-mammalian exendin-4. Data from an immunogenicity study in mice support the absence of any clinically relevant immunogenicity of the CM1 peptide.

Sequence listing

HAEGTFTSDV SSYLEGQAAK EFIAWLVKGR G
Sequence ID No.:1

RRDFPEEVAI VEELG
Sequence ID No.:2

RRDFPEEVAI AEELG
Sequence ID No.:3

HADGSFSDEM NTILDNLAAR DFINWLIQTK ITDRK
Sequence ID No.:4

HADGSFSDEM STILDNLATR DFINWLIQTK ITDKK
Sequence ID No.:5

HAEGTFTSDV SSYLEGQAAK EFIAWLVKGR GRRDFPEEVA IAEELGRRHA
EGTFTSDVSS YLEGQAAKEF IAWLVKGRG
Sequence ID No.:6

HAEGTFTSDV SSYLEGQAAK EFIAWLVKGR GRRDFPEEVA IAEELGRRHA
DGSFSDEMST ILDNLATRDF INWLIQTKIT DKK
Sequence ID No.:7

HAEGTFTSDV SSYLEGQAAK EFIAWLVKGR GRRDFPEEVA IAEELG
Sequence ID No.:8

MAPAAWLRSA AARALLPPML LLLLQPPPLL ARALPPDVHH LHAERRGPQP
WHAALPSSPA PAPATQEAPR PASSLRPPRC GVPDPSDGLS ARNRQKR
Sequence ID No.:9

HAEGTFTSDV SSYLEGQAAK EFIAWLVKGR GRRDFPEEVA IVEELGRRHA
EGTFTSDVSS YLEGQAAKEF IAWLVKGRG
Sequence ID No.:10

HAEGTFTSDV SSYLEGQAAK EFIAWLVKGR GRRDFPEEVA IVEELGRRHA
DGSFSDEMNT ILDNLAARDF INWLIQTKIT DRK
Sequence ID No.:11

HAEGTFTSDV SSYLEGQAAK EFIAWLVKGR GRRDFPEEVA IVEELG
Sequence ID No.:12

1 gatatccacc atggcccccg ccgcctggct gaggagcgcc gccgccaggg
M A P A A W L R S A A A R A
51 ccctgctgcc acccatgctg ctgctgctgc tgcagccccc acctctgctg
L L P P M L L L L Q P P P L L
101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L
401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggccgg cgacacgccg agggcacctt cacctccgac gtgagcagct
L G R R H A E G T F T S D V S S Y
751 acctggaggg ccaggccgcc aaggagttca tcgcctggct ggtgaagggc
L E G Q A A K E F I A W L V K G
801 aggggctgag cgcgc
R G *

Sequence ID No.:13

1 gatatccacc atggcccccg ccgcctggct gaggagcgcc gccgccaggg ccctgctgcc
61 acccatgctg ctgctgctgc tgcagccccc acctctgctg gcccgggccc tgcccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
661 gccgcaggga cttccctgag gaggtggcca tcgtggagga gctgggccgg cgacacgccg
721 agggcacctt cacctccgac gtgagcagct acctggaggg ccaggccgcc aaggagttca
781 tcgcctggct ggtgaagggc aggggctgag cgcgc

Sequence ID No.:14

 1   gatatccacc atggcccccg ccgcctggct gaggagcgcc gccgccaggg
                M  A  P  A   A  W  L   R  S  A   A  A  R  A
51   ccctgctgcc acccatgctg ctgctgctgc tgcagccccc acctctgctg
      L  L  P   P  M  L   L  L  L   Q  P  P   P  L  L
101  gcccgggccc tgcccccggt gagtgcccgc cactcgccgt ccgctcctcg
      A  R  A  L   P  P
151  ctgagggggc gccgggcacg cgggctgggc ccagcggcgt atccggacgc
201  caagaaacca gagagccagc cagatgccaa agggccctgc catgtgccgg
251  tgccctttcc ctctccattt gccctgccac acagtgggct ggggttgcac
301  gtgtgtttgc tgacaggcca catctctaac tgtgggccat gtggacctta
351  ggcctgacca gaccctcatg tcttcctcct tcccaggacg tgcaccacct
                                                D  V   H  H  L
401  gcacgccgag aggcgcggcc ctcagccctg gcacgccgcc ctgccaagca
      H  A  E   R  R  G  P   Q  P  W   H  A  A   L  P  S  S
451  gccctgcccc tgccccagcc acccaggagg cccccaggcc tgccagcagc
      P  A  P   A  P  A   T  Q  E  A   P  R  P   A  S  S
501  ctgaggccac ccaggtgcgg cgtgcctgat ccctccgatg gcctgagcgc
      L  R  P  P   R  C  G   V  P  D   P  S  D  G   L  S  A
551  tcggaatcgg cagaagaggc acgccgaggg caccttcacc tccgacgtga
      R  N  R   Q  K  R  H   A  E  G   T  F  T   S  D  V  S
601  gcagctacct ggagggccag gccgccaagg agttcatcgc ctggctggtg
      S  Y  L   E  G  Q   A  A  K  E   F  I  A   W  L  V
651  aagggcaggg gccgcaggga cttccctgag gaggtggcca tcgtggagga
      K  G  R  G   R  R  D   F  P  E   E  V  A  I   V  E  E
701  gctgggccgg cgacacgccg acggcagctt cagcgacgag atgaacacca
      L  G  R   R  H  A  D   G  S  F   S  D  E   M  N  T  I
751  tcctggacaa cctggccgcg cgcgacttca tcaactggct gatccagacc
      L  D  N   L  A  A   R  D  F  I   N  W  L   I  Q  T
801  aagatcaccg atcggaagtg agcgcgctga tatc
      K  I  T  D   R  K  *

Sequence ID No.:15

  1  gatatccacc atggcccccg ccgcctggct gaggagcgcc gccgccaggg ccctgctgcc
 61  acccatgctg ctgctgctgc tgcagccccc acctctgctg gcccgggccc tgcccccggt
121  gagtgcccgc cactcgccgt ccgctcctcg ctgagggggc gccgggcacg cggctgggc
181  ccagcggcgt atccggacgc caagaaacca gagagccagc cagatgccaa agggccctgc
241  catgtgccgg tgccctttcc ctctccattt gccctgccac acagtgggct ggggttgcac
301  gtgtgtttgc tgacaggcca catctctaac tgtgggccat gtggacctta ggcctgacca
361  gaccctcatg tcttcctcct tcccaggacg tgcaccacct gcacgccgag aggcgcggcc
421  ctcagccctg gcacgccgcc ctgccaagca gccctgcccc tgccccagcc acccaggagg
481  cccccaggcc tgccagcagc ctgaggccac ccaggtgcgg cgtgcctgat ccctccgatg
541  gcctgagcgc tcggaatcgg cagaagaggc acgccgaggg caccttcacc tccgacgtga
601  gcagctacct ggagggccag gccgccaagg agttcatcgc ctggctggtg aagggcaggg
661  gccgcaggga cttccctgag gaggtggcca tcgtggagga gctgggccgg cgacacgccg
721  acggcagctt cagcgacgag atgaacacca tcctggacaa cctggccgcg cgcgacttca
781  tcaactggct gatccagacc aagatcaccg atcggaagtg agcgcgctga tatc

Sequence ID No.:16

 1   gatatccacc atggcccccg ccgcctggct gaggagcgcc gccgccaggg
                M  A  P  A   A  W  L   R  S  A   A  A  R  A
51   ccctgctgcc acccatgctg ctgctgctgc tgcagccccc acctctgctg
      L  L  P   P  M  L   L  L  L   Q  P  P   P  L  L

101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L
401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggccgg cgacacgccg acggcagctt cagcgacgag atgaacacca
L G R R H A D G S F S D E M N T I
751 tcctggacaa cctggccgcg cgctga tat c
L D N L A A R *

Sequence ID No.:17

1 gatatccacc atggcccccg ccgcctggct gaggagcgcc gccgccaggg ccctgctgcc
61 acccatgctg ctgctgctgc tgcagccccc acctctgctg gcccgggccc tgccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
721 acggcagctt cagcgacgag atgaacacca tcctggacaa cctggccgcg cgctgatatc

Sequence ID No.:18

1 gatatccacc atggcccccg ccgcctggct gaggagcgcc gccgccaggg
M A P A A W L R S A A A R A
51 ccctgctgcc acccatgctg ctgctgctgc tgcagccccc acctctgctg
L L P P M L L L L Q P P P L L
101 gcccgggccc tgcccccggt gagtgcccgc cactcgccgt ccgctcctcg
A R A L P P
151 ctgagggggc gccgggcacg cgggctgggc ccagcggcgt atccggacgc
201 caagaaacca gagagccagc cagatgccaa agggccctgc catgtgccgg
251 tgccctttcc ctctccattt gccctgccac acagtgggct ggggttgcac
301 gtgtgtttgc tgacaggcca catctctaac tgtgggccat gtggacctta
351 ggcctgacca gaccctcatg tcttcctcct tcccaggacg tgcaccacct
D V H H L

401 gcacgccgag aggcgcggcc ctcagccctg gcacgccgcc ctgccaagca
H A E R R G P Q P W H A A L P S S
451 gccctgcccc tgccccagcc acccaggagg cccccaggcc tgccagcagc
P A P A P A T Q E A P R P A S S
501 ctgaggccac ccaggtgcgg cgtgcctgat ccctccgatg gcctgagcgc
L R P P R C G V P D P S D G L S A
551 tcggaatcgg cagaagaggc acgccgaggg caccttcacc tccgacgtga
R N R Q K R H A E G T F T S D V S
601 gcagctacct ggagggccag gccgccaagg agttcatcgc ctggctggtg
S Y L E G Q A A K E F I A W L V
651 aagggcaggg gccgcaggga cttccctgag gaggtggcca tcgtggagga
K G R G R R D F P E E V A I V E E
701 gctgggctga gcgcgc
L G *

Sequence ID No.:19

1 gatatccacc atggcccccg ccgcctggct gaggagcgcc gccgccaggg ccctgctgcc
61 acccatgctg ctgctgctgc tgcagccccc acctctgctg gcccgggccc tgcccccggt
121 gagtgcccgc cactcgccgt ccgctcctcg ctgagggggc gccgggcacg cgggctgggc
181 ccagcggcgt atccggacgc caagaaacca gagagccagc cagatgccaa agggccctgc
241 catgtgccgg tgccctttcc ctctccattt gccctgccac acagtgggct ggggttgcac
301 gtgtgtttgc tgacaggcca catctctaac tgtgggccat gtggacctta ggcctgacca
361 gaccctcatg tcttcctcct tcccaggacg tgcaccacct gcacgccgag aggcgcggcc
421 ctcagccctg gcacgccgcc ctgccaagca gccctgcccc tgccccagcc acccaggagg
481 cccccaggcc tgccagcagc ctgaggccac ccaggtgcgg cgtgcctgat ccctccgatg
541 gcctgagcgc tcggaatcgg cagaagaggc acgccgaggg caccttcacc tccgacgtga
601 gcagctacct ggagggccag gccgccaagg agttcatcgc ctggctggtg aagggcaggg
661 gccgcaggga cttccctgag gaggtggcca tcgtggagga gctgggctga gcgcgc

Sequence ID No.:20

HGEGTFTSDV SSYLEGQAAK EFIAWLVKGR G
Sequence ID No.:21

RRDFPEEVAI
Sequence ID No.:22

RRDFPEEVAI VEEL
Sequence ID No.:23

RRDFPEEVAI AEEL
Sequence ID No.:24

Asp Phe Pro Glu Glu Val Ala

Sequence ID No: 25

Ala Ala Asp Phe Pro Glu Glu Val Ala Ile

Sequence ID No: 26

Ala Ala Asp Phe Pro Glu Glu Val Ala Ile Val Glu Glu Leu

Sequence ID No: 27

Ala Ala Asp Phe Pro Glu Glu Val Ala Ile Ala Glu Glu Leu

Sequence ID No: 28

Ala Ala Asp Phe Pro Glu Glu Val Ala Ile Val Glu Glu Leu Gly

Sequence ID No: 29

Ala Ala Asp Phe Pro Glu Glu Val Ala Ile Ala Glu Glu Leu Gly

Sequence ID No: 30

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Phe Pro Glu Glu Val Ala Ile Ala Glu Glu Leu Gly

Sequence ID No: 31

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Ala Glu Glu Val Ala Ile Ala Glu Glu Leu Gly

Sequence ID No:32

His Ala Glu Gly Thr Phe Thr Ser Asp Val Ser Ser Tyr Leu Glu Gly

Gln Ala Ala Lys Glu Phe Ile Ala Trp Leu Val Lys Gly Arg Gly Arg
Arg Asp Ala Ala Ala Ala Val Ala Ile Ala Glu Glu Leu Gly

Sequence ID No: 33

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Ala Ala Ala Ala Val Ala Ile Ala Ala Ala Leu Gly

Sequence ID No: 34

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro

Sequence ID No: 35

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro Glu Glu Val Ala

Sequence ID No: 36

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro Glu Glu Val Ala Ile Ala Glu Glu Leu Gly Arg Arg
His Ala Cys

Sequence ID No: 37

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Phe Pro Glu Glu Val Ala Ile Val Glu Glu Leu Gly

Sequence ID No: 38

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Ala Glu Glu Val Ala Ile Val Glu Glu Leu Gly

Sequence ID No: 39

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Ala Ala Ala Ala Val Ala Ile Val Glu Glu Leu Gly

Sequence ID No: 40

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Ala
Ala Asp Ala Ala Ala Ala Val Ala Ile Val Ala Ala Leu Gly

Sequence ID No: 41

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Gly Arg
Arg Asp Phe Pro Glu Glu Val Ala Ile Val Glu Glu Leu Gly Arg Arg
His Ala Cys

Sequence ID No: 42

His Ala Glu Gly Thr Phe Thr Ser Asp Val Ser Ser Tyr Leu Glu Gly
Gln Ala Ala Lys Glu Phe Ile Ala Trp Leu Val Lys Gly Arg Xaa

Sequence ID No: 43 (formula (I))

Xaa Xaa Glu Gly Thr Phe Thr Ser Asp Xaa Ser Xaa Xaa Xaa Glu Xaa
Xaa Ala Xaa Xaa Xaa Phe Ile Xaa Trp Leu Xaa Xaa Xaa Xaa Xaa

Sequence ID No: 44 (formula (II))

Xaa Xaa Glu Gly Thr Phe Thr Ser Asp Val Ser Xaa Tyr Leu Glu Xaa
Xaa Ala Ala Xaa Glu Phe Ile Xaa Trp Leu Val Xaa Xaa Xaa Xaa

Sequence ID No: 45 (formula (III))

## Claims

**1.** The fusion peptide comprising as component (I) N-terminally a sequence according to formula II
Xaa7-XaaB-Glu-Gly-Thr-Phe-Thr-Ser-Asp-Xaa 16-Ser-Xaa 18-Xaa 19-Xaa20-Glu-Xaa22-Xaa23-Ala-Xaa25-Xaa26-Xaa27-Phe-Ile-Xaa3o-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, lie, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is is Ser, Lys or Arg; Xaa19 is Tyr or Gln ; Xaa20 is Leu or Met; Xaa22 is Gly, Glu or Aib; Xaa23 is Gln, Glu, Lys or Arg ; Xaa25 is Ala or Val ; Xaa26 is Lys, Glu or Arg; Xaa27 is Glu or Leu; Xaa30 isAla, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent.
or formula III
Xaa7-XaaB-Glu-Gly-Thr-Phe-Thr-Ser-Asp-Val-Ser-XaaB-Tyr-Leu-Glu-Xaa22-Xaa23-Ala-Ala-Xaa26-Glu-Phe-lle-Xaa30- Trp-Leu-Val-Xaa34-Xaa35-Xaa36-Xaa37,
wherein Xaa7 is L-histidine, D-histidine, desamino-histidine, 2-amino-histidine, - hydroxy-histidine, homohistidine, N-acetyl-histidine, a-fluoromethyl-histidine, a-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg ; Xaa26 is Lys, Glu or Arg;Xaa30 isAla, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent.
and as component (II) C-terminally a peptide sequence of at least 9 amino acids according to general formula (A)
-NH₂-(X1_{b}-Y1ₐ-X2_{b}-Y2a-X3_{b})_{c}-COOH (A)
whereby
Y1 and Y2 is a negatively charged amino acid as described above,
X1, X2 and X3 is any amino acid except for a negatively charged amino acid a and b are from 0 to 15, whereby a may be identical or different for X1, X2, and X3, and b may be different or identical for Y1 and Y2, and
c is from 1 to 5,
wherein,
if Y2 is E (glutamate) and a is 2 for Y2,
(i) a is not 0 or 2 for Y1, if Y1 is E, or (ii) X2 (for b >= 1) contains any amino acid apart from V, A or P (valin, alanin or prolin) as residue being neighboured to E of Y2, or (iii) X3 (for b >= 1) contains any amino acid apart from L or V (leucin or valin) as residue being neighboured to E of Y2, or (iv) c is not 1, if Y1 is E and a is 2 for Y1.

**2.** The fusion peptide according to claim 1 comprising as component (I) N-terminally a GLP-1(7-35, 7-36 or 7-37) sequence or a functional fragment, variant or derivative thereof.

**3.** The fusion peptide according to claim 1 comprising as component (I) N-terminally a sequence according to formula I
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-X (I), wherein X is NH2 or Gly-OH,
or a functional fragment, variant or derivative thereof.

**4.** A fusion peptide according to claim 2 or 3, wherein component (I) contains a sequence having at least 80 % sequence homology with SEQ ID No.: 1.

**5.** A fusion peptide according to claims 1 to 4, wherein component (II) is a peptide sequence forming a β-turn like structure.

**6.** A fusion peptide according to any of preceding claims 1 to 5, wherein component (II) is a peptide sequence containing at least one alanine or proline residue.

**7.** A fusion peptide according to any of preceding claims 1 to 6, wherein component (II) is a peptide sequence containing a tetramer with β turn forming properties, e.g. having a proline residue at position 2 of that tetramer.

**8.** A fusion peptide according to any of preceding claims 1 to 7, wherein component (II) is a peptide sequence containing a sequence motif selected from the group consisting of DFP(EE)A, DFPEEI, DFP(EE)L, DFPEET, DFP(EE)S, DFPEEG, P(EE)SAI, P(EE)TAI, P(EE)LAI, PEEIAI, P(EE)AAI, PEEGAI, G(EE)VAI, TEEVAI, A(EE)VAI, LEEVAI, S(EE)VAI, MEEVAI, F(EE)VAI, IAEEA, IA(EE)T, IAEES, IA(EE)G, IAEEP, IA(EE)V, IAEEM, IA(EE)W, IA(EE)Y, or, IA(EE)F, whereby the core motif (EE). All of the afore-mentioned motifs may exhibit, instead of the (EE) core may be exchanged by (DD), (DE), (ED), (EED), (EEE), (EDE), (DEE), (DDD), (DDE), (DED), (EDD), (DDDD), (EEEE), (EDEE), (EEDE), (EEED), (DDDE), (DDEE).

**9.** A fusion peptide according to any of preceding claims 1 to 8, wherein component (II) is a peptide sequence being linked to the C-terminus of component (I) by its N-terminal sequence motif selected from the group consisting of AA, XA, AX, RR, RX, and XR.

**10.** A fusion peptide according to any of preceding claims 1 to 9, wherein component (II) is a peptide sequence containing the sequence motif having more than 20%, more preferred more than 30%, more preferred more than 40% and even more preferred more than 50%, and, simultaneously, less than 80% sequence homology to the sequence motif SEQ ID No.: 25 (DFPEEVA), whereby typically the amino acid P and/or the amino acid V of that sequence motif are substituted by any other amino acid sequence.

**11.** A fusion peptide according to any of preceding claims 1 to 10, wherein component (II) is a peptide sequence containing a sequence motif selected from the group consisting of motifs having more than 20%, more preferred more than 30%, more preferred more than 40% and even more preferred more than 50%, and, simultaneously, less than 85%, preferably less than 80% sequence homology to SEQ ID No.: 22 (RRDFPEEVAI) or SEQ ID No.: 26 (AADFPEEVAI), whereby the amino acid P and/or the amino acid V of sequence ID No. 22 or SEQ ID No. 26 are substituted by any other amino acid .

**12.** A fusion peptide according to any of preceding claims 1 to 11, wherein component (II) is a peptide sequence containing a sequence motif selected from a group consisting of motifs having more than 20%, more preferred more than 30%, more preferred more than 40% and even more preferred more than 50%, and, simultaneously, less than 95 or less than 90%, more preferably less than 85%, even more preferably less than 80% sequence homology to SEQ ID No.: 23 (RRDFPEEVAIVEEL)) or SEQ ID No. 24 (RRDFPEEVAIAEEL)), SEQ ID No.: 27 (AADFPEEVAIVEEL), and SEQ ID No.: 28 (AADFPEEVAIAEEL), whereby the amino acid A (at position 11 of SEQ. ID No. 24), P, L and/or the amino acid V (at position 8 of both sequences) of sequence ID Nos. 23, 24, 27, 28 are substituted by any other amino acid.

**13.** A fusion peptide according to any of preceding claims 1 to 12, wherein component (II) is a peptide sequence containing a sequence motif selected from the group consisting of motifs having at least 20%, more preferred more than 30%, more preferred more than 40% and even more preferred more than 50%, and, simultaneously, less than 95% or less than 90%, more preferably less than 85%, even more preferably less than 80% sequence homology to SEQ ID No.: 2 (RRDFPEEVAIVEELG), SEQ ID No. 3 (RRDFPEEVAIAEELG), SEQ ID No.: 29 (AADFPEEVAIVEELG), and SEQ ID NO.: 30 (AADFPEEVAIAEELG), whereby the amino acid A, V (at position 11 of SEQ. ID No. 3 or 30 or 2 or 29, respectively), P, L and/or the amino acid V (at position 8 of all above sequences) are substituted by any other amino acid.

**14.** A fusion peptide according to any of preceding claims 1 to 13, wherein component (II) is a peptide sequence having 9 to 30, preferably 9 to 20, and most preferably 9 to 15 amino acids.

**15.** A fusion peptide according to any of preceding claims 1 to 14, wherein component (I) and component (II) are directly linked or linked via a linker sequence.

**16.** A fusion peptide according to claim 15, wherein the linker sequence has a length of 1 to 10 amino acids.

**17.** A fusion peptide according to any of preceding claims 1 to 16, wherein the fusion peptide contains another component (III) linked to the C-terminus of component (II) and/or to the N-terminus of component (I).

**18.** A fusion peptide according to claim 17, wherein component (III) comprises at least four amino acid residues, preferably at least 10 additional amino acid residues, more preferably at least 20, or at least 30.

**19.** A fusion peptide according to claim 17 or 18, wherein component (III) comprises at least 4, preferably at least 10, more preferably at least 20 additional amino acid residues of the N-terminal sequence of GLP-2 as in proglucagon or of GLP-1 (7-37) .

**20.** A fusion peptide according to any of claims 17 to 19, wherein component (III) contains the sequence of SEQ ID Nos.: 4 or 5 or a sequence having at least 80% sequence homology with SEQ ID Nos.: 4 or 5.

**21.** A fusion peptide according to any of claims 17 to 20, wherein the fusion peptide contains a peptide sequence selected from a group consisting of: SEQ ID No. 6, SEQ ID No. 7, SEQ ID No. 10 and SEQ ID No. 11 or a sequence having at least 80% sequence homology with SEQ ID Nos.: 6, 7, 10, or 11.

**22.** A fusion peptide according to any of preceding claims 1 to 21, wherein at least one of the amino acids is derivatized by a covalent modification of a side chain of a naturally occurring amino acid, by a modification of the peptide backbone, by modification of the NH₂ or carboxy terminal groups.

**23.** A fusion peptide according to claim 22, wherein at least one of the amino acids is derivatized by a lipyl or carbohydrate group.

**24.** A fusion peptide according to claim 22 or 23, wherein the N-terminal His residue of GLP-1 (GLP-1(7)) is chemically modified at its NH2 terminus and/or at its histidyl side chain, in particular by a hydrophobic moiety.

**25.** A fusion peptide according to any of preceding claims 1 to 24, wherein the fusion peptide comprises a carrier protein, in particular transferrin or albumin, as component (IV).

**26.** A fusion peptide according to any of preceding claims 1 to 25, wherein the amino acid sequence of components (I), (II) and/or (III) is reversed and wherein said amino acid sequence(s) is at least partially composed of D amino acid isomers.

**27.** Method of producing a fusion peptide according to any of preceding claims 1 to 26 by solid state peptide synthesis.

**28.** A nucleic acid encoding a fusion peptide according to any of preceding claims 1 to 21 or 25.

**29.** A vector comprising a nucleic acid according to claim 28.

**30.** A host cell comprising exogenously introduced DNA according to claim 28, in particular a vector according to claim 29, being capable of expressing said fusion peptide.

**31.** Method for producing a fusion peptide according to any of preceding claims 1 to 21 or 25 in which a micro-organism transformed to include a nucleic acid encoding the fusion peptide is fermented and the protein is recovered.

**32.** A method of producing a protein according to claim 31 in which animal cells are grown under conditions in which the protein is exported from the cells.

**33.** Fusion peptide according to any of preceding claims 1 to 26 as a medicament for use in human or animal therapy.

**34.** Use of a fusion peptide according to any of preceding claims 1 to 26, a nucleic acid according to claim 28, a vector according to claim 29 or a host cell according to claim 30 for the manufacture of a medicament for the treatment of diabetes mellitus type I or type II, insulin resistance, weight disorders and diseases or conditions associated thereto.

**35.** Use of a fusion peptide according to any of preceding claims 1 to 26, a nucleic acid according to claim 28, a vector according to claim 29 or a host cell according to claim 30 for the manufacture of a medicament for the treatment of neurodegenerative disorders and diseases or conditions associated thereto.

**37.** Use of a fusion peptide according to any of preceding claims 1 to 26, a nucleic acid according to claim 28, a vector according to claim 29 or a host cell according to claim 30 for the manufacture of a medicament for the treatment of disorders and diseases or conditions associated to apoptosis.


SEQUENCE LISTING

<110>  Biocompatibles UK Limited

<120>  Novel GLP-1 fusion peptides, their production and use

<130>  BI07P005EP

<140>  EP07006321.9
<141>  2007-03-27

<160>  45

<170>  PatentIn version 3.3

<210>  1
<211>  31
<212>  PRT
<213>  Artificial

<220>
<223>  Description of sequence:  synthetic peptide corresponding to
       GLP-1(7-37) (see description p. 4 and 48)

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  15
<212>  PRT
<213>  Artificial

<220>
<223>  Description of sequence: full-length IP-2 sequence having all 15
       amino acids of the naturally occurring IP-2 sequence, human;
       (see description p. 8 and 48)

<400>  2

Arg Arg Asp Phe Pro Glu Glu Val Ala Ile Val Glu Glu Leu Gly
1               5                   10                  15

<210>  3
<211>  15
<212>  PRT
<213>  Artificial

<220>
<223>  Description of sequence: full-length IP-2 sequence having all 15
       amino acids of the naturally occurring IP-2 sequence, murine;
       (see description p. 8 and 48)

<400>  3

Arg Arg Asp Phe Pro Glu Glu Val Ala Ile Ala Glu Glu Leu Gly

1               5                   10                  15

<210>  4
<211>  35
<212>  PRT
<213>  Artificial

<220>
<223>  Description of sequence: murine isoform of GLP-2
       (see description p. 12 and 48)

<400>  4

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp Arg Lys
        35

<210>  5
<211>  35
<212>  PRT
<213>  Artificial

<220>
<223>  Description of sequence: human isoform of GLP-2
       (see description p. 12 and 48)

<400>  5

His Ala Asp Gly Ser Phe Ser Asp Glu Met Ser Thr Ile Leu Asp Asn
1               5                   10                  15

Leu Ala Thr Arg Asp Phe Ile Asn Trp Leu Ile Gln Thr Lys Ile Thr
            20                  25                  30

Asp Lys Lys
        35

<210>  6
<211>  79
<212>  PRT
<213>  Artificial

<220>
<223>  Description of sequence: SEQ ID No: 6 (ID6syn, CM1) corresponds
       to GLP-1(7-37)-IP2-RR-GLP1(7-37), 79 aa, 8,7 kD
       (see description p. 13 and 48)

<400>  6

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  7
<211>  83
<212>  PRT
<213>  Artificial

<220>
<223>  Description of sequence: SEQ ID No: 7 (ID7rec, CM2) corresponds
       to GLP-1(7-37)-IP2-RR-GLP2, 83 aa, 9,4 kD
       (see description p. 13 and 48)

<400>  7

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Asp Gly Ser Phe Ser Asp Glu Met Ser Thr Ile Leu Asp Asn
    50                  55                  60

Leu Ala Thr Arg Asp Phe Ile Asn Trp Leu Ile Gln Thr Lys Ile Thr
65                  70                  75                  80

Asp Lys Lys

<210>  8
<211>  46
<212>  PRT
<213>  Artificial

<220>
<223>  Description of sequence: SEQ ID No:8 (ID8 syn, CM3) corresponds
       to GLP-1(7-37)-IP2, 46 aa, 5,1 kD;(see description p. 9 and 48)

<400>  8

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly
        35                  40                  45

<210>  9
<211>  97
<212>  PRT
<213>  Artificial

<220>
<223>  Description of sequence: artificial (see description p. 48)

<400>  9

Met Ala Pro Ala Ala Trp Leu Arg Ser Ala Ala Ala Arg Ala Leu Leu
1               5                   10                  15

Pro Pro Met Leu Leu Leu Leu Leu Gln Pro Pro Pro Leu Leu Ala Arg
            20                  25                  30

Ala Leu Pro Pro Asp Val His His Leu His Ala Glu Arg Arg Gly Pro
        35                  40                  45

Gln Pro Trp His Ala Ala Leu Pro Ser Ser Pro Ala Pro Ala Pro Ala
    50                  55                  60

Thr Gln Glu Ala Pro Arg Pro Ala Ser Ser Leu Arg Pro Pro Arg Cys
65                  70                  75                  80

Gly Val Pro Asp Pro Ser Asp Gly Leu Ser Ala Arg Asn Arg Gln Lys
                85                  90                  95

Arg

<210>  10
<211>  79
<212>  PRT
<213>  Artificial

<220>
<223>  Description of sequence: SEQ ID No: 10
       (N-GLP-1(7-37)-IP2(human)-RR-GLP-1(7-37)-C, also designated human
       CM1), (see description p. 13 and 48)

<400>  10

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  11
<211>  83
<212>  PRT
<213>  Artificial

<220>
<223>  Description of sequence: SEQ ID No: 11
       (N-GLP-1(7-37)-IP2(human)-RR-GLP-2-C), also designated human CM2
       herein), (see description p. 13 and 48)

<400>  11

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Asp Gly Ser Phe Ser Asp Glu Met Asn Thr Ile Leu Asp Asn
    50                  55                  60

Leu Ala Ala Arg Asp Phe Ile Asn Trp Leu Ile Gln Thr Lys Ile Thr
65                  70                  75                  80

Asp Arg Lys

<210>  12
<211>  46
<212>  PRT
<213>  Artificial

<220>
<223>  Description of sequence: SEQ ID No: 12, GLP-1(7-37) linked
       without any linker sequence via its C-terminus to human IP2
       (see description p. 9 and 48)

<400>  12

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly
        35                  40                  45

<210>  13
<211>  815
<212>  DNA
<213>  Artificial

<220>
<223>  Description of sequence: (SEQ ID No:13) represents the
       translated peptide sequence of the construct according to Fig. 1k
       (SEQ ID No: 14), (see description p. 37 and 49)

<220>
<221>  CDS
<222>  (11)..(118)

<220>
<221>  CDS
<222>  (387)..(809)

<400>  13
gatatccacc atg gcc ccc gcc gcc tgg ctg agg agc gcc gcc gcc agg        49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg       97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct         148
Leu Ala Arg Ala Leu Pro Pro
30                  35

cgctgagggg gcgccgggca cgcgggctgg gcccagcggc gtatccggac gccaagaaac    208

cagagagcca gccagatgcc aaagggccct gccatgtgcc ggtgcccttt ccctctccat    268

ttgccctgcc acacagtggg ctggggttgc acgtgtgttt gctgacaggc cacatctcta    328

actgtgggcc atgtggacct taggcctgac cagaccctca tgtcttcctc cttcccag      386

gac gtg cac cac ctg cac gcc gag agg cgc ggc cct cag ccc tgg cac      434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc      482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat      530
Pro Arg Pro Ala Ser Ser Leu Arg Pro Pro Arg Cys Gly Val Pro Asp

70                  75                  80

ccc tcc gat ggc ctg agc gct cgg aat cgg cag aag agg cac gcc gag        578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc        626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc        674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gag        722
Pro Glu Glu Val Ala Ile Val Glu Glu Leu Gly Arg Arg His Ala Glu
        135                 140                 145

ggc acc ttc acc tcc gac gtg agc agc tac ctg gag ggc cag gcc gcc        770
Gly Thr Phe Thr Ser Asp Val Ser Ser Tyr Leu Glu Gly Gln Ala Ala
    150                 155                 160

aag gag ttc atc gcc tgg ctg gtg aag ggc agg ggc tga gcgcgc             815
Lys Glu Phe Ile Ala Trp Leu Val Lys Gly Arg Gly
165                 170                 175

<210>  14
<211>  815
<212>  DNA
<213>  Artificial

<220>
<223>  Description of sequence: DNA sequence of the construct according
       to Fig. 1k, (see description p. 37 and 49)

<400>  14
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

agggcacctt cacctccgac gtgagcagct acctggaggg ccaggccgcc aaggagttca     780

tcgcctggct ggtgaagggc aggggctgag cgcgc                                815

<210>  15
<211>  834
<212>  DNA
<213>  Artificial

<220>
<223>  Description of sequence: DNA sequence and sequence of the
       translated peptide according to the construct Fig. 1h, (see
       description p. 37 and 50)

<220>
<221>  CDS
<222>  (11)..(118)

<220>
<221>  CDS
<222>  (387)..(821)

<400>  15
gatatccacc atg gcc ccc gcc gcc tgg ctg agg agc gcc gcc gcc agg       49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg      97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct        148
Leu Ala Arg Ala Leu Pro Pro
30                  35

cgctgagggg gcgccgggca cgcgggctgg gcccagcggc gtatccggac gccaagaaac   208

cagagagcca gccagatgcc aaagggccct gccatgtgcc ggtgcccttt ccctctccat   268

ttgccctgcc acacagtggg ctggggttgc acgtgtgttt gctgacaggc cacatctcta   328

actgtgggcc atgtggacct taggcctgac cagaccctca tgtcttcctc cttcccag     386

gac gtg cac cac ctg cac gcc gag agg cgc ggc cct cag ccc tgg cac     434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc     482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat     530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag     578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc     626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc     674

Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac      722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg      770
Gly Ser Phe Ser Asp Glu Met Asn Thr Ile Leu Asp Asn Leu Ala Ala
    150                 155                 160

cgc gac ttc atc aac tgg ctg atc cag acc aag atc acc gat cgg aag      818
Arg Asp Phe Ile Asn Trp Leu Ile Gln Thr Lys Ile Thr Asp Arg Lys
165                 170                 175                 180

tga gcgcgctgat atc                                                   834

<210>  16
<211>  834
<212>  DNA
<213>  Artificial

<220>
<223>  Description of sequence: DNA sequence of the construct according
       to Fig. 1h, (see description p. 37 and 50)

<400>  16
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

acggcagctt cagcgacgag atgaacacca tcctggacaa cctggccgcg cgcgacttca     780

tcaactggct gatccagacc aagatcaccg atcggaagtg agcgcgctga tatc           834

<210>  17
<211>  780
<212>  DNA
<213>  Artificial

<220>
<223>  Description of sequence: DNA sequence sequence and translated

peptide sequence of the construct according to Fig. 11,
(see description p. 37 and 50)

<220>
<221> CDS
<222> (11)..(118)

<220>
<221> CDS
<222> (387)..(776)

<400> 17
gatatccacc atg gcc ccc gcc gcc tgg ctg agg agc gcc gcc gcc agg 49
Met Ala Pro Ala Ala Trp Leu Arg Ser Ala Ala Ala Arg
1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac 722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg 770
Gly Ser Phe Ser Asp Glu Met Asn Thr Ile Leu Asp Asn Leu Ala Ala
    150                 155                 160

cgc tga tatc                                                          780
Arg
165


<210>  18
<211>  720
<212>  DNA
<213>  Artificial

<220>
<223>  Description of sequence: DNA sequence of the construct according
       to Fig. 1l, (see description p. 37 and 51)

<400>  18
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

acggcagctt cagcgacgag atgaacacca tcctggacaa cctggccgcg cgctgatatc     720


<210>  19
<211>  716

<212>  DNA
<213>  Artificial

<220>
<223>  Description of sequence: DNA sequence and sequence of the
       translated peptide according to Fig. 1m., (see description p.
       37 and 51)

<220>
<221>  CDS
<222>  (11)..(118)

<220>
<221>  CDS
<222>  (387)..(710)

<400>  19
gatatccacc atg gcc ccc gcc gcc tgg ctg agg agc gcc gcc gcc agg         49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc tga gcgcgc 716
Pro Glu Glu Val Ala Ile Val Glu Glu Leu Gly
        135                 140

<210> 20
<211> 716
<212> DNA
<213> Artificial

<220>
<223> Description of sequence: DNA sequence of the construct
according to Fig. 1m, (see description p. 37 and 52)

<400> 20
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

gccgcaggga cttccctgag gaggtggcca tcgtggagga gctgggctga gcgcgc 716

<210> 21
<211> 31
<212> PRT
<213> Artificial

<220>
<223> Description of sequence: artificial, (see description p. 52)

<400> 21

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 22
<211> 10
<212> PRT
<213> Artificial

<220>
<223> Description of sequence: artificial, (see description p. 8 and 52)

<400> 22

Arg Arg Asp Phe Pro Glu Glu Val Ala Ile
1               5                   10

<210> 23
<211> 14
<212> PRT
<213> Artificial

<220>
<223> Description of sequence: artificial, (see description p. 8 and 52)

<400> 23

Arg Arg Asp Phe Pro Glu Glu Val Ala Ile Val Glu Glu Leu
1               5                   10

<210>  24
<211>  14
<212>  PRT
<213>  Artificial

<220>
<223>  Description of sequence: artificial, (see description p. 8 and 52)

<400>  24

Arg Arg Asp Phe Pro Glu Glu Val Ala Ile Ala Glu Glu Leu
1               5                   10

<210>  25
<211>  7
<212>  PRT
<213>  Artificial

<220>
<223>  Description of sequence: partial sequence derived from IP2
        (see description p. 8 and 53)

<400>  25

Asp Phe Pro Glu Glu Val Ala
1               5

<210>  26
<211>  10
<212>  PRT
<213>  Artificial

<220>
<223>  Description of sequence: sequence derived from SEQ ID NO: 22,
       wherein the first two arginines have been replaced by two alanines
        (see description p. 8 and 53)

<400>  26

Ala Ala Asp Phe Pro Glu Glu Val Ala Ile
1               5                   10

<210>  27
<211>  14
<212>  PRT
<213>  Artificial

<220>
<223>  Description of sequence: sequence derived from SEQ ID NO: 23,
       wherein the first two arginines have been replaced by two
       alanines (see description p. 8 and 53)

<400>  27

Ala Ala Asp Phe Pro Glu Glu Val Ala Ile Val Glu Glu Leu
1               5                   10

<210>  28
<211>  14

<212>  PRT
<213>  Artificial

<220>
<223>  Description of sequence: sequence derived from SEQ ID NO: 24,
       wherein the first two arginines have been replaced by two
       alanines(see description p. 9 and 53)

<400>  28

Ala Ala Asp Phe Pro Glu Glu Val Ala Ile Ala Glu Glu Leu
1               5                   10


<210>  29
<211>  15
<212>  PRT
<213>  Artificial

<220>
<223>  Description of sequence: sequence derived from SEQ ID NO: 2,
       wherein the first two arginines have been replaced by two
       alanines (see description p. 9 and 53)

<400>  29

Ala Ala Asp Phe Pro Glu Glu Val Ala Ile Val Glu Glu Leu Gly
1               5                   10                  15


<210>  30
<211>  15
<212>  PRT
<213>  Artificial

<220>
<223>  Description of sequence: sequence derived from SEQ ID NO: 3,
       wherein the first two arginines have been replaced by two
       alanines (see description p. 9 and 53)

<400>  30

Ala Ala Asp Phe Pro Glu Glu Val Ala Ile Ala Glu Glu Leu Gly
1               5                   10                  15


<210>  31
<211>  46
<212>  PRT
<213>  Artificial

<220>
<223>  Description of sequence: sequence derived from GLP-1-IP2-fragment
       (murine) (see description p. 9 and 53)

<400>  31

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly Ala
            20                  25                  30

Ala Asp Phe Pro Glu Glu Val Ala Ile Ala Glu Glu Leu Gly
        35                  40                  45

<210>  32
<211>  46
<212>  PRT
<213>  Artificial

<220>
<223>  Description of sequence: sequence derived from GLP-1-IP2-fragment
       (murine) (see description p. 9 and 53)

<400>  32

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Ala Glu Glu Leu Gly
        35                  40                  45

<210>  33
<211>  46
<212>  PRT
<213>  Artificial

<220>
<223>  Description of sequence: sequence derived from GLP-1-IP2-fragment
       (murine) (see description p. 9 and 53)

<400>  33

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Ala Glu Glu Leu Gly
        35                  40                  45

<210>  34
<211>  46
<212>  PRT
<213>  Artificial

<220>
<223>  Description of sequence: sequence derived from GLP-1-IP2-fragment
       (murine) (see description p. 9 and 54)

<400>  34

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Ala Ala Ala Leu Gly
        35                  40                  45

<210>  35
<211>  36
<212>  PRT
<213>  Artificial

<220>
<223>  Description of sequence: sequence derived from GLP-1-IP2-fragment
       (murine) (see description p. 9 and 54)

<400>  35

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro
        35

<210>  36
<211>  40
<212>  PRT
<213>  Artificial

<220>
<223>  Description of sequence: sequence derived from GLP-1-IP2-fragment
       (murine) (see description p. 9 and 54)

<400>  36

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala
        35                  40

<210>  37
<211>  51
<212>  PRT
<213>  Artificial

<220>
<223>  Description of sequence: sequence derived from GLP-1-IP2-fragment
       (murine) (see description p. 9 and 54)

<400>  37

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Cys
    50

<210>  38
<211>  46
<212>  PRT
<213>  Artificial

<220>
<223>  Description of sequence: sequence derived from GLP-1-IP2-fragment
       (human) (see description p. 9 and 54)

<400>  38

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Phe Pro Glu Glu Val Ala Ile Val Glu Glu Leu Gly
        35                  40                  45

<210>  39
<211>  46
<212>  PRT
<213>  Artificial

<220>
<223>  Description of sequence: sequence derived from GLP-1-IP2-fragment
       (human) (see description p. 9 and 54)

<400>  39

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Val Glu Glu Leu Gly
        35                  40                  45

<210>  40
<211>  46
<212>  PRT
<213>  Artificial

<220>
<223>  Description of sequence: sequence derived from GLP-1-IP2-fragment
       (human) (see description p. 9 and 55)

<400>  40

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Val Glu Glu Leu Gly
        35                  40                  45

<210>  41
<211>  46
<212>  PRT
<213>  Artificial

<220>
<223>  Description of sequence: sequence derived from GLP-1-IP2-fragment
       (human) (see description p. 9 and 55)

<400>  41

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Val Ala Ala Leu Gly
        35                  40                  45

<210>  42
<211>  51
<212>  PRT
<213>  Artificial

<220>
<223>  Description of sequence: sequence derived from GLP-1-IP2-fragment
       (human) (see description p. 9 and 55)

<400>  42

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Cys
    50

<210>  43
<211>  31
<212>  PRT
<213>  Artificial

<220>
<223>  Description of sequence: sequence according to formula (I)
       (see description, p. 1 and 55)

<220>
<221>  misc_feature
<222>  (31)..(31)
<223>  Xaa = NH2, when sequence is GLP-1(7-36) amide, or Xaa = Gly-OH,
       when sequence is GLP-1(7-37) amide

<400>  43

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Xaa
            20                  25                  30

<210>  44
<211>  31
<212>  PRT
<213>  Artificial

<220>
<223>  Description of sequence:  sequence according to formula (II)
       (see description p. 19 and 55)

<220>
<221>  VARIATION
<222>  (1)..(1)
<223>  /replace="D-histidine"
       /replace="desamino-histidine"
       /replace="2-amino-histidine"
       /replace="3-hydroxy-histidine"
       /replace="homohistidine"
       /replace="N-acetyl-histidine"
       /replace="alpha-fluoromethyl-histidine"
       /replace="alpha-methyl-histidine"
       /replace="3-pyridylalanine"
       /replace="2-pyridylalanine"
       /replace="4-pyridylalanine"

<220>
<221>  VARIATION
<222>  (2)..(2)

<223>  /replace="Ala"
       /replace="Val"
       /replace="Leu"
       /replace="Ile"
       /replace="Lys"
       /replace="Aib"
       /replace="(1-aminocyclopropyl) carboxylic acid"
       /replace="(1-aminocyclobutyl) carboxylic acid"
       /replace="(1-aminocyclopentyl) carboxylic acid"
       /replace="(1-aminocyclohexyl) carboxylic acid"
       /replace="(1-aminocycloheptyl) carboxylic acid"
       /replace="(1-aminocyclooctyl) carboxylic acid"

<220>
<221>  VARIATION
<222>  (10)..(10)
<223>  /replace="Leu"

<220>
<221>  VARIATION
<222>  (12)..(12)
<223>  /replace="Lys"
       /replace="Arg"

<220>
<221>  VARIATION
<222>  (13)..(13)
<223>  /replace="Gln"

<220>
<221>  VARIATION
<222>  (14)..(14)
<223>  /replace="Met"

<220>
<221>  VARIATION
<222>  (16)..(16)
<223>  /replace="Glu"
       /replace="Aib"

<220>
<221>  VARIATION
<222>  (17)..(17)
<223>  /replace="Glu"
       /replace="Lys"
       /replace="Arg"

<220>
<221>  VARIATION
<222>  (19)..(19)
<223>  /replace="Val"

<220>
<221>  VARIATION
<222>  (20)..(20)
<223>  /replace="Glu"
       /replace="Arg"

<220>
<221>  VARIATION
<222>  (21)..(21)
<223>  /replace="Leu"

<220>

<221>  VARIATION
<222>  (24)..(24)
<223>  /replace="Glu"
       /replace="Arg"

<220>
<221>  VARIATION
<222>  (27)..(27)
<223>  /replace="Lys"

<220>
<221>  VARIATION
<222>  (28)..(28)
<223>  /replace="Glu"
       /replace="Asn"
       /replace="Arg"

<220>
<221>  VARIATION
<222>  (29)..(29)
<223>  /replace="Aib"

<220>
<221>  VARIATION
<222>  (30)..(30)
<223>  /replace="Gly"
       /replace="Lys"
       /replace="amide"
       /replace=""

<220>
<221>  VARIATION
<222>  (31)..(31)
<223>  /replace="Ala"
       /replace="Glu"
       /replace="Pro"
       /replace="Lys"
       /replace="amide"
       /replace=""

<400>  44

His Gly Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly
            20                  25                  30


<210>  45
<211>  31
<212>  PRT
<213>  Artificial

<220>
<223>  Description of sequence: sequence according to formula (III)
       (see description p. 19 and 55)

<220>
<221>  VARIATION
<222>  (1)..(1)
<223>  /replace="D-histidine"
       /replace="desamino-histidine"

/replace="2-amino-histidine"
/replace="3-hydroxy-histidine"
/replace="homohistidine"
/replace="N-acetyl-histidine"
/replace="alpha-fluoromethyl-histidine"
/replace="alpha-methyl-histidine"
/replace="3-pyridylalanine"
/replace="2-pyridylalanine"
/replace="4-pyridylalanine"

<220>
<221>  VARIATION
<222>  (2)..(2)
<223>  /replace="Gly"
       /replace="Val"
       /replace="Leu"
       /replace="Ile"
       /replace="Lys"
       /replace="Aib"
       /replace="(1-aminocyclopropyl) carboxylic acid"
       /replace="(1-aminocyclobutyl) carboxylic acid"
       /replace="(1-aminocyclopentyl) carboxylic acid"
       /replace="(1-aminocyclohexyl) carboxylic acid"
       /replace="(1-aminocycloheptyl) carboxylic acid"
       /replace="(1-aminocyclooctyl) carboxylic acid"

<220>
<221>  VARIATION
<222>  (12)..(12)
<223>  /replace="Lys"
       /replace="Arg"

<220>
<221>  VARIATION
<222>  (16)..(16)
<223>  /replace="Glu"
       /replace="Aib"

<220>
<221>  VARIATION
<222>  (17)..(17)
<223>  /replace="Glu"
       /replace="Lys"
       /replace="Arg"

<220>
<221>  VARIATION
<222>  (20)..(20)
<223>  /replace="Glu"
       /replace="Arg"

<220>
<221>  VARIATION
<222>  (24)..(24)
<223>  /replace="Glu"
       /replace="Arg"

<220>
<221>  VARIATION
<222>  (28)..(28)
<223>  /replace="Glu"
       /replace="Arg"

<220>

<221>  VARIATION
<222>  (29)..(29)
<223>  /replace="Aib"

<220>
<221>  VARIATION
<222>  (30)..(30)
<223>  /replace="Lys"
       /replace="amide"
       /replace=""

<220>
<221>  VARIATION
<222>  (31)..(31)
<223>  /replace="Ala"
       /replace="Glu"
       /replace="Lys"
       /replace="amide"
       /replace=""

<400>  45

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30